(19) Europäisches Patentamt
European Patent Office
Office européen des brevets

(11) Veröffentlichungsnummer: **0 456 068 B1**

# (12) EUROPÄISCHE PATENTSCHRIFT

(45) Veröffentlichungstag der Patentschrift: **02.08.95**

(21) Anmeldenummer: **91106892.2**

(22) Anmeldetag: **27.04.91**

(51) Int. Cl.[6]: **C07D 307/14**, C07C 251/34, C07D 333/04, C07D 317/28, C07D 339/06, C07D 309/04, C07D 309/20, C07D 257/02, C07C 239/08, A01N 43/08, A01N 43/10

## (54) Cyclohexenonoximether, Verfahren und Zwischenprodukte zu ihrer Herstellung und ihre Verwendung als Herbizide.

(30) Priorität: **09.05.90 DE 4014984**

(43) Veröffentlichungstag der Anmeldung:
**13.11.91 Patentblatt 91/46**

(45) Bekanntmachung des Hinweises auf die Patenterteilung:
**02.08.95 Patentblatt 95/31**

(84) Benannte Vertragsstaaten:
**AT BE CH DE ES FR GB IT LI NL**

(56) Entgegenhaltungen:
**EP-A- 0 125 094**
**EP-A- 0 218 233**
**US-A- 4 440 566**

(73) Patentinhaber: **BASF Aktiengesellschaft**
**Carl-Bosch-Strasse 38**
**D-67063 Ludwigshafen (DE)**

(72) Erfinder: **Kast, Juergen, Dr.**
**Kastanienstrasse 24**
**W-6737 Boehl-Iggelheim (DE)**
Erfinder: **Meyer, Norbert, Dr.**
**Dossenheimer Weg 22**
**W-6802 Ladenburg (DE)**
Erfinder: **Misslitz, Ulf, Dr.**
**Am Herzel 40**
**W-6730 Neustadt (DE)**
Erfinder: **Harreus, Albrecht, Dr.**
**Teichgasse 13**
**W-6700 Ludwigshafen (DE)**
Erfinder: **Kuekenhoehner, Thomas, Dr.**
**Seidelstrasse 2**
**W-6710 Frankenthal (DE)**
Erfinder: **Rang, Harald, Dr.**
**Maximilianstrasse 30**
**W-6700 Ludwigshafen (DE)**
Erfinder: **Gerber, Matthias, Dr.**
**Ritterstrasse 3**
**W-6704 Mutterstadt (DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Rank Xerox (UK) Business Services
(3.10/3.09/3.3.3)

Erfinder: **Westphalen, Karl-Otto, Dr.**
**Mausbergweg 58**
**W-6720 Speyer (DE)**
Erfinder: **Walter, Helmut, Dr.**
**Gruenstadter Strasse 82**
**W-6719 Obrigheim (DE)**

## Beschreibung

Die Erfindung betrifft neue herbizid wirksame Cyclohexenonoximether der Formel I

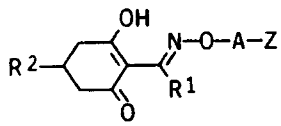

(I)

in der die variablen folgende Bedeutung haben:

$R^1$ eine $C_1$-$C_6$-Alkylgruppe;

A eine unsubstituierte oder durch 1 bis 3 $C_1$-$C_3$-Alkylgruppen, Halogenatome substituierte $C_3$-$C_6$-Alkinylenkette;

Z Phenyl, 5-gliedriger oder 6-gliedriger Heteroaromat mit einem bis drei Heteroatomen, ausgewählt aus einer Gruppe bestehend aus drei Stickstoffatomen und einem Sauerstoff- oder Schwefelatom, wobei die aromatischen Reste unsubstituiert oder mit n gleichen oder verschiedenen Resten X substituiert sein können;

X Nitro, Cyano, Halogen, $C_1$-$C_4$-Alkyl, $C_1$-$C_4$-Alkoxy, $C_1$-$C_4$-Alkylthio, $C_1$-$C_4$-Halogenalkyl, $C_1$-$C_4$-Halogenalkoxy, Carboxyl, $C_1$-$C_4$-Alkoxycarbonyl, Benzyloxycarbonyl, Phenyl, wobei die aromatischen Reste noch einen bis drei Substituenten tragen können, ausgewählt aus einer Gruppe bestehend aus Nitro, Cyano, Halogen, $C_1$-$C_4$-Alkyl, $C_1$-$C_4$-Alkoxy, $C_1$-$C_4$-Alkylthio, $C_1$-$C_4$-Halogenalkyl, $C_1$-$C_4$-Halogenalkoxy, Carboxyl, $C_1$-$C_4$-Alkoxycarbonyl- und Benzyloxycarbonyl;

n 0 bis 3 oder 1 bis 5 für den Fall, daß X Halogenatome bedeutet;

$R^2$ eine $C_1$-$C_4$-Alkoxy-$C_1$-$C_6$-alkyl- oder $C_1$-$C_4$-Alkylthio-$C_1$-$C_6$-alkylgruppe;

eine $C_3$-$C_7$-Cycloalkylgruppe oder eine $C_5$-$C_7$-Cycloalkenylgruppe, wobei diese Gruppen noch einen bis drei Reste tragen können, ausgewählt aus einer Gruppe bestehend aus $C_1$-$C_4$-Alkyl, $C_1$-$C_4$-Alkoxy, $C_1$-$C_4$-Alkylthio, $C_1$-$C_4$-Halogenalkyl, Hydroxy und Halogen;

ein 5-gliedriger gesättigter Heterocyclus der ein oder zwei Heteroatome enthält, ausgewählt aus einer Gruppe bestehend aus Sauerstoff und Schwefel, und der noch einen bis drei Reste tragen kann, ausgewählt aus einer Gruppe bestehend aus $C_1$-$C_4$-Alkyl, $C_1$-$C_4$-Alkoxy, $C_1$-$C_4$-Alkylthio und $C_1$-$C_4$-Halogenalkyl,

ein 6- oder 7-gliedriger gesättigter oder ein- oder zweifach ungesättigter Heterocyclus, enthaltend ein oder zwei Heteroatome, ausgewählt aus einer Gruppe bestehend aus Sauerstoff und Schwefel, wobei der Heterocyclus noch einen bis drei Reste tragen kann, ausgewählt aus einer Gruppe bestehend aus Hydroxy, Halogen, $C_1$-$C_4$-Alkyl, $C_1$-$C_4$-Alkoxy, $C_1$-$C_4$-Alkylthio und $C_1$-$C_4$-Halogenalkyl;

ein 5-gliedriger Heteroaromat, enthaltend ein bis drei Heteroatome, ausgewählt aus einer Gruppe bestehend aus zwei Stickstoffatomen und einem Sauerstoff- oder Schwefelatom, wobei der Heterocyclus noch einen bis drei Reste tragen kann, ausgewählt aus einer Gruppe bestehend aus Halogen, Cyano, $C_1$-$C_4$-Alkyl, $C_1$-$C_4$-Alkoxy. $C_1$-$C_4$-Alkylthio, $C_1$-$C_4$-Halogenalkyl, $C_2$-$C_6$-Alkenyl, $C_2$-$C_6$-Alkenyloxy, $C_2$-$C_6$-Halogenalkenyl und $C_1$-$C_4$-Alkoxy-$C_1$-$C_4$-alkyl,

die Phenylgruppe oder die Pyridylgruppe, wobei diese Gruppen noch einen bis drei Reste tragen können, ausgewählt aus einer Gruppe bestehend aus Halogen, Nitro, Cyano, $C_1$-$C_4$-Alkyl, $C_1$-$C_4$-Alkoxy, $C_1$-$C_4$-Alkylthio, $C_1$-$C_4$-Halogenalkyl, $C_3$-$C_6$-Alkenyloxy, $C_3$-$C_6$-Alkinyloxy und $-NR^3R^4$, worin

$R^3$ Wasserstoff, eine $C_1$-$C_4$-Alkylgruppe, eine $C_3$-$C_6$-Alkenylgruppe oder eine $C_3$-$C_6$-Alkinylgruppe und

$R^4$ Wasserstoff, eine $C_1$-$C_4$-Alkylgruppe, eine $C_3$-$C_6$-Alkenylgruppe, eine $C_3$-$C_6$-Alkinylgruppe, eine $C_1$-$C_6$-Acylgruppe oder ein Benzoylrest, wobei der aromatische Ring zusätzlich einen bis drei Substituenten tragen kann, ausgewählt aus einer Gruppe bestehend aus Nitro, Cyano, Halogen, $C_1$-$C_4$-Alkyl, $C_1$-$C_4$-Alkoxy, $C_1$-$C_4$-Alkylthio und $C_1$-$C_4$-Halogenalkyl

bedeuten,

sowie ihre landwirtschaftlich nutzbaren Salze und Ester von $C_1$-$C_{10}$-Carbonsäuren und anorganischen Säuren.

Außerdem betrifft die Erfindung ein Verfahren und Zwischenprodukte zu ihrer Herstellung sowie ihre Anwendung als Pflanzenschutzmittel.

Die erfindungsgemäßen Cyclohexenone I haben offensichtlich sauren Charakter, d.h. sie können einfache Umsetzungsprodukte wie Salze von Alkali- oder Erdalkaliverbindungen oder Enolester bilden.

Die Verbindungen der Formel I können in mehreren tautomeren Formen auftreten, die alle vom Anspruch erfaßt werden.

In der Literatur sind Cyclohexenone der allgemeinen Formel I'

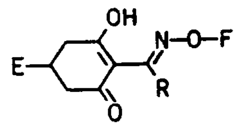

I'

in der u.a.

a) F Benzyl und E 2-Ethylthiopropyl (US-A 4 440 566);

b) F Benzyl, But-2-enyl und E einen substituierten 5-gliedrigen Heteroarylrest (EP-A 238 021, EP-A 125 094);

c) F Benzyl, But-2-enyl und E ein substituiertes Phenyl (EP-A 80 301);

d) F But-2-enyl und E einen 5- bis 7-gliedrigen heterocyclischen Ring mit bis zu zwei O-, S-Atomen und mit bis zu zwei Doppelbindungen (EP-A 218 233)

e) F 4-Phenylbutyl, 4-Phenyl-but-2-enyl, 4-Phenylbut-3-enyl und E die unter a) bis d) aufgeführten Reste (ältere deutsche Anmeldung P 38 38 309)

bedeuten, als Herbizide beschrieben.

Es werden jedoch Verbindungen gesucht, die bei geringer Aufwandmenge hohe Selektivität aufweisen, d.h. unerwünschte Pflanzen bekämpfen, ohne dabei die Kulturpflanzen zu schädigen.

Entsprechend dieser Aufgabe wurden die neuen Cyclohexenonoximether der Formel I gefunden, die sich durch eine gute herbizide Wirkung gegen unerwünschte Gräser auszeichnen. Die Verbindungen sind mit breitblättrigen Kulturpflanzen sowie einige mit Gramineenkulturen wie Reis verträglich.

Die Cyclohexenone der Formel I können in an sich bekannter Weise aus schon bekannten Derivaten der Formel II (EP-A 80 301, EP-A-125 094, EP-A 142 741, US-A 4 249 937, EP-A 137 174 und EP-A 177 913) und den entsprechenden Hydroxylaminen der Formel III (Houben-Weyl, 10/1 S. 1181 ff) hergestellt werden (EP-A 169 521).

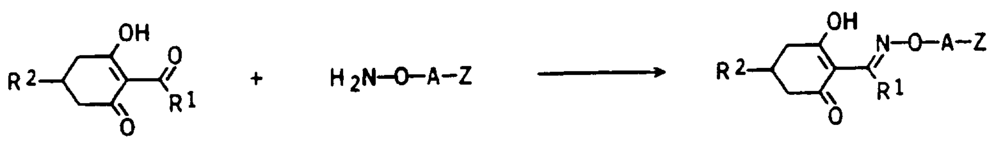

II III I

Zweckmäßig führt man die Umsetzung in heterogener Phase in einem Lösungsmittel, bei einer ausreichenden Temperatur unterhalb von etwa 80 °C, in Gegenwart einer Base durch und verwendet das Hydroxylamin III in Form seines Ammoniumsalzes.

Geeignete Basen sind z. B. Carbonate, Hydrogencarbonate, Acetate, Alkoholate oder Oxide von Alkali- oder Erdalkalimetallen, insbesondere Natriumhydroxid, Kaliumhydroxid, Magnesiumoxid, Calciumoxid. Außerdem können organische Basen wie Pyridin oder tertiäre Amine Verwendung finden. Die Base wird beispielsweise in einer Menge von 0,5 bis 2 Mol-Äquivalent bezogen auf die Ammoniumverbindung zugesetzt.

Als Lösungsmittel eignen sich beispielsweise Dimethylsulfoxid; Alkohole wie Methanol, Ethanol und Isopropanol; aromatische Kohlenwasserstoffe wie Benzol und Toluol; chlorierte Kohlenwasserstoffe wie Chloroform und Dichlorethan; aliphatische Kohlenwasserstoffe wie Hexan und Cyclohexan; Ester wie Essigsäureethylester und Ether wie Diethylether, Dioxan und Tetrahydrofuran. Vorzugsweise führt man die Umsetzung in Methanol mit Natriumhydrogencarbonat als Base durch.

Die Reaktion ist nach wenigen Stunden beendet. Die Zielverbindung kann z.B. durch Einengen der Mischung, Verteilung des Rückstandes in Methylenchlorid/Wasser und Abdestillieren des Lösungsmittels unter vermindertem Druck isoliert werden.

Man kann für diese Umsetzung aber auch unmittelbar die freie Hydroxylaminbase, z.B. in Form einer wäßrigen Lösung, verwenden; je nach verwendetem Lösungsmittel für die Verbindung II erhält man ein- oder zweiphasiges Reaktionsgemisch.

Geeignete Lösungsmittel für diese variante sind beispielsweise Alkohole wie Methanol, Ethanol, Isopropanol und Cyclohexanol; aliphatische und aromatische, gegebenenfalls chlorierte Kohlenwasserstoffe wie Hexan, Cyclohexan, Methylenchlorid, Toluol und Dichlorethan; Ester wie Essigsäureethylester; Nitrile wie Acetonitril und cyclische Ether wie Dioxan und Tetrahydrofuran.

Alkalimetallsalze der Verbindungen I können durch Behandeln der 3-Hydroxyverbindungen mit Natrium- oder Kaliumhydroxid bzw. -alkoholat in wäßriger Lösung oder in einem organischen Lösungsmittel wie Methanol, Ethanol, Aceton und Toluol erhalten werden.

Andere Metallsalze wie Mangan-, Kupfer-, Zink-, Eisen-, Calcium-, Magnesium- und Bariumsalze können aus den Natriumsalzen in üblicher Weise hergestellt werden, ebenso Ammonium- und Phosphoniumsalze mittels Ammoniak, Phosphonium-, Sulfonium- oder Sulfoxoniumhydroxiden.

Die Verbindungen des Typs II können z.B. aus den entsprechenden Cyclohexan-1,3-dionen der Formel VII

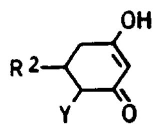 VII,

in der

Y Wasserstoff oder Methoxycarbonyl bedeutet

nach bekannten Methoden (Tetrahedron Lett., 2491 (1975)) hergestellt werden.

Es ist auch möglich, die Verbindungen der Formel II über die Zwischenstufe der Enolester VIII herzustellen, die bei der Umsetzung von Verbindungen der Formel VII mit Säurechloriden IX in Gegenwart von Basen anfallen und anschließend mit bestimmten Imidazol- oder Pyridinderivaten umgelagert werden (JP-OS 79/063 052).

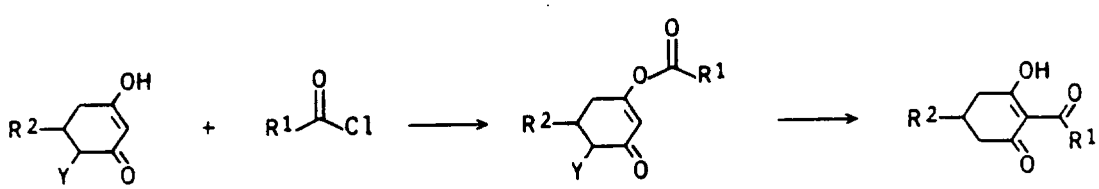

VII IX VIII II

Zu den Verbindungen der Formel VII gelangt man über eine Reihe bekannter Verfahrensschritte, ausgehend von bekannten Vorprodukten.

Die Synthese der Hydroxylamine III, in denen A eine substituierte oder unsubstituierte $C_3$-$C_6$-Alkinylenbrücke bedeutet, erfolgt gemäß dem nachstehenden Reaktionsschema nach literaturbekannten Verfahren (J. Med. Chem. 29, 1389 (1986); EP-A 131 302; J. Med. Chem. 24, 678 (1981); J. Chem. Ecol. 10, 1201 (1982)). Ausgehend von Aryl- oder Hetarylhalogeniden X durch Kupplung mit einem 1,ω-Alkinol XI, XIa in Gegenwart von Palladiumkatalysatoren (vgl. Tetrahedron Lett. 50 (1975) 4467). Das so erhaltene Alkinol IVa wird mit einem cyclischen Hydroxyimid V gekoppelt.

Die Verknüpfung kann direkt nach der Mitsunobu-Variante (Synthesis 1981, 1; J. Med. Chem. 33, 187 (1990)) zwischen dem Arylalkinol IVa und dem cyclischen Hydroxyimid V erfolgen oder durch Überführung der OH-Gruppe des Arylalkinols IVa in eine Abgangsgruppe X (z.B. Halogen, O-Mesylat, ...) und anschließender Substitution der Abgangsgruppe durch das Hydroxyimid V zu den Imidethern VI.

Verbindungen, bei denen die Dreifachbindung nicht in Konjugation zum Aromaten bzw. Heteroaromaten steht, können z.B. durch Reaktion eines Arylalkylhalogenids XII mit dem Dianion eines 1,ω-Alkinols XI zum Arylalkinol IVc umgesetzt werden. Das Arylalkinol IVc kann dann wieder direkt oder über die Zwischenstufe IVd zum geschützten Hydroxylaminderivat XIb, wie oben beschrieben, umgesetzt werden.

Das geschützte Hydroxylaminderivat VI wird mit einer Base, z.B. mit 2-Aminoethanol zum freien Hydroxylamin III gespalten:

Reaktionsschema:

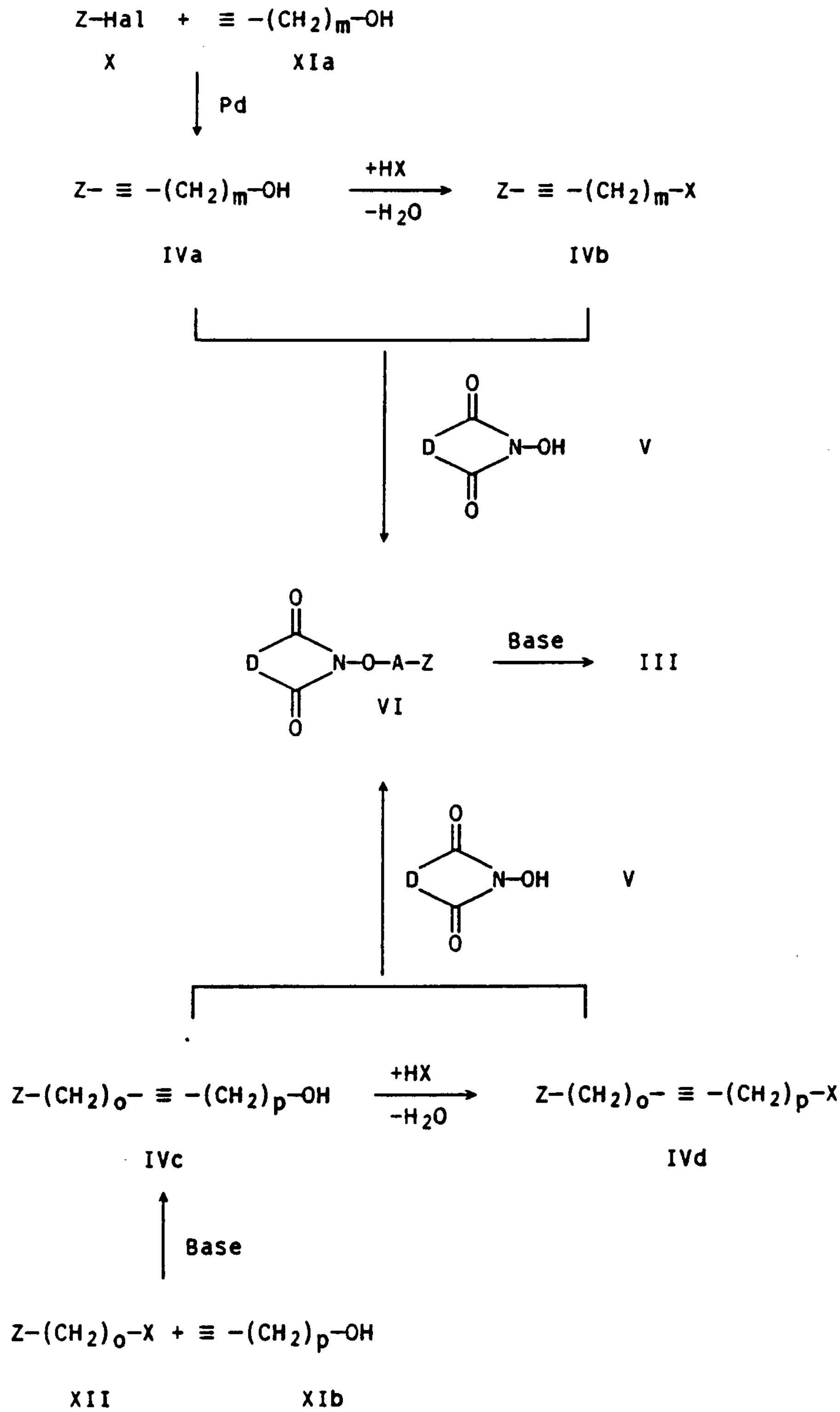


mit Hal = Cl, Br, I; X = Cl, Br, Mesylat, Tosylat; m = 1, 2, 3, 4; o, p = 1, 2, 3;
A = $C_3$-$C_6$-Alkinylen

In den cyclischen Hydroxyimiden steht D z.B. für Phenylen, Naphthylen, Pyridinylen, Cyclopentylen, Cyclohexylen oder Cyclohexenylen. Beispielsweise kommen folgende Substanzen in Betracht:

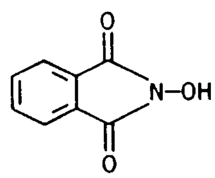
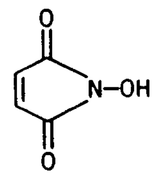
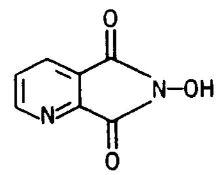

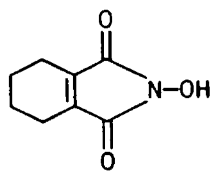
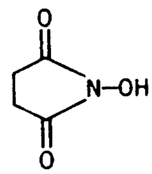
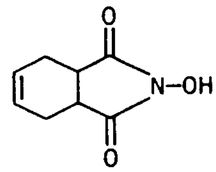

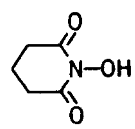

Die Umsetzung der Verbindungen IVb und IVd mit den Hydroxyimiden V wird zweckmäßigerweise in Gegenwart einer Base ausgeführt. Geeignet sind prinzipiell alle Basen, die in der Lage sind, die Hydroxyimide V zu deprotonieren ohne das Imidsystem anzugreifen. Dies sind insbesondere die sogenannten nicht-nucleophilen Basen. Beispielsweise genannt seien Mineralbasen wie Alkalimetall- und Erdalkalimetallcarbonate, Alkalimetall- und Erdalkalimetallhydrogencarbonate, organische Basen wie aliphatische, cycloaliphatische und aromatische tertiäre Amine. Es können auch Gemische dieser Basen verwendet werden.

Als Einzelverbindungen seien folgende Basen beispielhaft aufgeführt: Natriumcarbonat, Kaliumcarbonat, Magnesiumcarbonat, Calciumcarbonat, Bariumcarbonat, die Hydrogencarbonate dieser Metalle, Trimethylamin, Triethylamin, Tributylamin, Ethyldiisopropylamin, N,N-Dimethylanilin, 4-N,N-Dimethylaminopyridin, Diazabicyclooctan, Diazabicycloundecan, N-Methylpiperidin, 1,4-Dimethylpiperazin, Pyridin, Chinolin, Bipyridin, Phenanthrolin. Bevorzugt sind die preiswerten Basen Natrium- und Kaliumcarbonat.

Die Base wird im allgemeinen in äquivalenten Mengen bis zu einem Überschuß von 5 Äquivalenten, bezogen auf das Hydroxyimid, zugegeben. Ein höherer Überschuß ist möglich, entbehrt aber zusätzliche Vorteile. Die Verwendung einer geringen Basenmenge ist ebenfalls möglich. Bevorzugt wird jedoch eine Basenmenge von 1 bis 3, insbesondere von 1 bis 2 Äquivalenten, bezogen auf das Hydroxyimid V eingesetzt.

Die Verwendung von nucleophilen Basen, z.B. Alkalimetall- und Erdalkalimetallhydroxiden, insbesondere Natrium- und Kaliumhydroxid, ist ebenfalls möglich. In diesem Falle ist es vorteilhaft, die Base in äquivalenten Mengen bezüglich des Hydroxyimids VI einzusetzen, um einem nucleophilen Angriff der Hydroxylionen auf die Carbonylfunktion der Imidgruppierung vorzubeugen.

Zweckmäßigerweise setzt man die Ausgangsverbindungen VI mit den Hydroxyimiden V in einem Lösungsmittel um, das sich unter den Reaktionsbedingungen inert verhält. Vorteilhafte Lösungsmittel sind z.B. polareaprotische Lösungsmittel wie Dimethylformamid, N-Methylpyrrolidon, Dimethylsulfoxid, Sulfolan und cyclische Harnstoffe. Die Lösungsmittelmenge ist im allgemeinen nicht kritisch.

Die Umsetzung der Ausgangsverbindungen IVb, d mit den Hydroxyimiden V kann auch unter Anwendung der Phasentransfer-Katalyse ausgeführt werden. In diesem Falle werden mit Wasser zwei Phasen bildende Lösungsmittel, bevorzugt Chlorkohlenwasserstoffe, eingesetzt. Als Phasentransferkatalysatoren eignen sich die üblicherweise zu solchen Zwecken verwendeten quartären Ammonium- und Phosphoniumsalze, Polyethylenglykole, Polyethylenglykolether und Kronenether, wie sie z.B. in Dehmlow et al., Phase Transfer Catalysis, S. 37 - 45 und S. 86 - 93, Verlag Chemie, Weinheim 1980, beschrieben sind. Die Phasentransferkatalysatoren werden zweckmäßigerweise in Mengen von 1 bis 10 Vol%, bevorzugt in Mengen von 3 bis 5 Vol%, bezogen auf das Volumen der Reaktionsmischung, eingesetzt.

Die Umsetzung der Ausgangsverbindungen IVb und IVd mit den Hydroxyimiden V wird im allgemeinen im Temperaturbereich zwischen 0 und 140 °C, bevorzugt zwischen 20 und 100 °C, insbesondere zwischen 40 und 80 °C, durchgeführt. Zweckmäßigerweise wird dabei so vorgegangen, daß man das Hydroxyimid V zusammen mit der Base im Lösungsmittel vorlegt und das Ausgangsmaterial VI zu dieser Lösung dosiert. Dabei kann es sich als günstig erweisen, wenn das Hydroxyimid bei einer tieferen Temperatur, bespielsweise bei 0 bis 50 °C, zugegeben und die Reaktionsmischung erst nach dieser Zugabe auf die eigentliche Reaktionstemperatur erhitzt wird.

Nach beendeter Reaktion wird die abgekühlte Reaktionsmischung zweckmäßigerweise mit Wasser versetzt, wobei sich die gebildeten Hydroxylaminderivate VI als kristalline Festkörper oder als Öle abscheiden. Die auf diese Weise erhaltenen Hydroxylaminderivate können, falls gewünscht, durch Umkristallisation oder durch Extraktion weiter gereinigt werden.

Bei der Umsetzung der Alkinole IVa oder IVc mit einem cyclischen Hydroxyimid der Formel V, nach Mitsunobu, entstehen ebenfalls die cyclischen Imidether der Formel VI.

Die Kopplung der Alkohole IVa, c an ein Hydroxyimid der Formel V erfolgt in Gegenwart eines Triarylphosphinderivates und eines Azodicarbonsäurediesters in einem inerten Lösungsmittel (J. Med. Chem. 33, 187 (1990)). Aus Kostengründen kommt als Hydroxyimid V bevorzugt Hydroxyphthalimid zum Einsatz.

Als Phosphinderivat kommt z.B. Triphenylphosphin zur Anwendung und als Azodicarbonsäurediester verwendet man bevorzugt den Diethylester.

Als Lösungsmittel eignen sich aprotische organische Lösungsmittel wie z.B. Diethylether, Tetrahydrofuran, Toluol und Essigester.

Die Hydroxylaminderivate VI können zwischengelagert werden oder sogleich in die Hydroxylaminderivate III mit freier Aminogruppe umgewandelt werden. Diese Umwandlung kann nach an sich bekannten Verfahren durchgeführt werden, wie sie beispielsweise in DE-A 36 15 973 und den darin zitierten Schriften beschrieben sind. Bevorzugt wird das Verfahren gemäß DE-A 36 15 973 angewandt, nach dem die Hydroxylaminderivate III mittels Ethanolamin freigesetzt werden. Die Freisetzung der Hydroxylaminderivate III mit Hilfe anderer Basen wie wäßrigen Mineralbasen, mit Aminen, Hydrazinen, Hydroxylaminen oder mittels wäßriger Säuren ist ebenfalls möglich.

Aus den nach diesen Verfahren erhaltenen Reaktionsgemischen können die Hydroxylaminderivate III mittels üblicher Aufarbeitungsmethoden isoliert werden, beispielsweise durch Extraktion oder durch Kristallisation. Zur Erhöhung der Kristallisationstendenz dieser Hydroxylaminderivate kann es oftmals förderlich sein, diese in ihre Salze mit Mineralsäuren oder organischen Säuren überzuführen. Dazu werden im allgemeinen verdünnte Lösungen dieser Säuren mit den Hydroxylaminderivaten umgesetzt, und zwar zweckmäßigerweise in äquivalenten Mengen. Die erhaltenen Hydroxylammoniumsalze können wie die Hydroxylaminderivate mit freier Aminogruppe direkt zu den Herbiziden der Formel I weiterverarbeitet werden oder auch, falls gewünscht, gelagert werden.

Im Hinblick auf die biologische Wirksamkeit werden Cyclohexenone der Formel I bevorzugt, in denen die Substituenten folgende Bedeutung haben:

$R^1$ Alkyl wie Methyl, Ethyl, Propyl, 1-Methylethyl, Butyl, 1-Methylpropyl, 2-Methylpropyl, 1,1-Dimethylethyl, Pentyl, 1-Methylbutyl, 2-Methylbutyl, 3-Methylbutyl, 1,1-Dimethylpropyl, 1,2-Dimethylpropyl, 2,2-Dimethylpropyl, 1-Ethylpropyl, Hexyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl und 1-Ethyl-2-methylpropyl, insbesondere Ethyl und Propyl;

A Alkinylen wie Prop-2-inylen, But-2-inylen, But-3-inylen, Pent-2-inylen, Pent-3-inylen, Pent-4-inylen, Hex-2-inylen, Hex-3-inylen, Hex-4-inylen, Hex-5-inylen, Pent-2-in-4-enylen, Pent-4-in-2-enylen, Hex-2-in-4-enylen, Hex-2-in-5-enylen, Hex-3-in-5-enylen, Hex-4-in-2-enylen, Hex-5-in-2-enylen, Hex-5-in-3-enylen bedeuten und durch 1 bis 3 Methyl- bzw. Ethylreste und/oder Fluor bzw. Chlor substituiert sein können; bei den ungesättigten Ketten können sowohl die cis- als auch die trans-Form auftreten. Insbesondere bevorzugt sind But-2-inylen, But-3-inylen und Prop-2-inylen;

Z Phenyl, Thiophen, Furan, Pyrrol, Thiazol, Oxazol, Imidazol, Isothiazol, Isoxazol, Pyrazol, Pyridin, Pyrimidin, Pyrazin, Pyridazin, Triazin sein. Bevorzugt sind Phenyl, Thiophen, Furan, Thiazol, Pyridin, Pyrimiden, besonders bevorzugt sind Phenyl, Thiophen und Pyridin.

X Halogen wie Fluor, Chlor, Brom und Iod, insbesondere Fluor und Chlor;

Alkyl wie Methyl, Ethyl, Propyl, 1-Methylethyl, Butyl, 1-Methylpropyl, 2-Methylpropyl und 1,1-Dimethylethyl, insbesondere Methyl und 1,1-Dimethylethyl,

Alkoxy wie Methoxy, Ethoxy, Propoxy, 1-Methylethoxy, Butoxy, 1-Methylpropoxy, 2-Methylpropoxy und 1,1-Dimethylethoxy, insbesondere Methoxy, Ethoxy, 1-Methylethoxy und 1,1-Dimethylethoxy,

Alkylthio wie Methylthio, Ethylthio, Propylthio, 1-Methylethylthio, Butylthio, 1-Methylpropylthio, 2-Methylpropylthio und 1,1-Dimethylethylthio, insbesondere Methylthio und Ethylthio,

Halogenalkyl wie Fluormethyl, Difluormethyl, Trifluormethyl, Chlordifluormethyl, Dichlorfluormethyl, Trichlormethyl, 1-Fluorethyl, 2-Fluorethyl, 2,2-Difluorethyl, 2,2,2-Trifluorethyl, 2-Chlor-2,2-difluorethyl, 2,2-Dichlor-2-fluorethyl, 2,2,2-Trichlorethyl und Pentafluorethyl, insbesondere Difluormethyl, Trifluormethyl, 2,2,2-Trifluorethyl und Pentafluorethyl,
Halogenalkoxy wie Difluormethoxy, Trifluormethoxy, Chlor-difluormethoxy, Dichlorfluormethoxy, 1-Fluorethoxy, 2-Fluorethoxy, 2,2-Difluorethoxy, 1,1,2,2-Tetrafluorethoxy, 2,2,2-Trifluorethoxy, 2-Chlor-1,1,2-trifluorethoxy und Pentafluorethoxy, insbesondere Trifluormethoxy,
Alkoxycarbonyl wie Methoxycarbonyl, Ethoxycarbonyl, Propyloxycarbonyl, 1-Methylethoxycarbonyl, Butyloxycarbonyl und 1,1-Dimethylethoxycarbonyl, insbesondere Methoxycarbonyl, Ethoxycarbonyl und 1,1-Dimethylethoxycarbonyl, insbesondere Methoxycarbonyl sowie
Nitro, Cyano,
Benzyloxycarbonyl und Phenyl, wobei die aromatischen Reste ihrerseits ein bis drei der folgenden Reste tragen können: Nitro, Cyano, Carboxyl, Benzyloxycarbonyl, Halogen wie im allgemeinen und im Besonderen bei X genannt; Alkyl wie bei $R^1$ genannt, insbesondere Methyl, Ethyl und 1-Methylethyl; Alkoxy wie vorstehend genannt, insbesondere Methoxy und Ethoxy; Alkylthio wie vorstehend genannt, insbesondere Methylthio; Halogenalkyl wie vorstehend genannt, insbesondere Trifluormethyl; Halogenalkoxy wie vorstehend genannt, insbesondere Difluormethoxy und Trifluormethoxy und/oder Alkoxycarbonyl wie vorstehend genannt, insbesondere Methoxycarbonyl und Ethoxycarbonyl.
Besonders bevorzugt sind bei diesen aromatischen Resten unsubstituierte oder einfach substituierte Vertreter.

n 0, 1, 2 oder 3, insbesondere 0, 1 und 2. Bei mehreren Resten X können die Substituenten gleich oder verschieden sein.

$R^2$ Alkyl wie unter $R^1$ genannt, welches eine der unter X genannten Alkoxy- oder Alkylthiogruppe bevorzugt in 1-, 2- oder 3-Position tragen kann, insbesondere 2-Ethylthiopropyl;
5-gliedriges Heterocycloalkyl wie Tetrahydrofuranyl, Tetrahydrothienyl, Dioxolanyl, Dithiolanyl und Oxathiolanyl, insbesondere Tetrahydrofuranyl, Tetrahydrothienyl und Dioxolanyl, wobei diese Ringe ein bis drei der bereits unter X genannten $C_1$-$C_4$-Alkylgruppen, $C_1$-$C_4$-Alkoxygruppen, $C_1$-$C_4$-Alkylthiogruppen und/oder $C_1$-$C_4$-Halogenalkylgruppen tragen können,
5-gliedriges Heteroaryl wie Pyrrolyl, Pyrazolyl, Imidazolyl, Isoxazolyl, Oxazolyl, Isothiazolyl, Thiazolyl, Furanyl und Thienyl, insbesondere Isoxazolyl und Furanyl,
ein 6- oder 7-gliedriger Heterocyclus wie Tetrahydropyran-3-yl, Dihydropyran-3-yl, Tetrahydropyran-4-yl, Dihydropyran-4-yl, Tetrahydrothiopyran-3-yl, Dihydrothiopyran-3-yl, Tetrahydrothiopyran-4-yl, Dihydrothiopyran-4-yl und Dioxepan-5-yl, insbesondere Tetrahydropyran-3-yl, Tetrahydropyran-4-yl und Tetrahydrothiopyran-3-yl,
ein Phenyl- oder ein Pyridylrest,
wobei die cyclischen Reste ein bis drei der unter X genannten Alkylgruppen, Alkoxygruppen, Alkylthiogruppen und/oder Halogenalkylgruppen tragen können.
Die 5-gliedrigen Heteroaromaten in der Bedeutung $R^2$ können als Substituenten folgende Reste tragen:
Halogenatom wie unter X genannt, insbesondere Fluor und Chlor,
Alkoxyalkyl wie Methoxymethyl, 2-Methoxyethyl, 2-Methoxypropyl, 3-Methoxypropyl, 2-Methoxy-1-methylethyl, Ethoxymethyl, 2-Ethoxyethyl, 2-Ethoxypropyl, 3-Ethoxypropyl, 2-Ethoxy-1-methylethyl und 1-Ethoxy-1-methylethyl, insbesondere Methoxyethyl und Ethoxyethyl,
Alkenyl wie Ethenyl, 1-Propenyl, 2-Propenyl, 1-Methylethenyl, 1-Butenyl, 2-Butenyl, 3-Butenyl, 1-Methyl-1-propenyl, 1-Methyl-2-propenyl, 2-Methyl-1-propenyl, 2-Methyl-2-propenyl, 1-Pentenyl, 2-Pentenyl, 3-Pentenyl, 4-Pentenyl, 1-Methyl-1-butenyl, 2-Methyl-1-butenyl, 3-Methyl-1-butenyl, 1-Methyl-2-butenyl, 2-Methyl-2-butenyl, 3-Methyl-2-butenyl, 1-Methyl-3-butenyl, 2-Methyl-3-butenyl, 3-Methyl-3-butenyl, 1,1-Dimethyl-2-propenyl, 1,2-Dimethyl-1-propenyl, 1,2-Dimethyl-2-propenyl, 1-Ethyl-1-propenyl, 1-Ethyl-2-propenyl, 1-Hexenyl, 2-Hexenyl, 3-Hexenyl, 4-Hexenyl, 5-Hexenyl, 1-Methyl-1-pentenyl, 2-Methyl-1-pentenyl, 3-Methyl-1-pentenyl, 4-Methyl-1-pentenyl, 1-Methyl-2-pentenyl, 2-Methyl-2-pentenyl, 3-Methyl-2-pentenyl, 4-Methyl-2-pentenyl, 1-Methyl-3-pentenyl, 2-Methyl-3-pentenyl, 3-Methyl-3-pentenyl, 4-Methyl-3-pentenyl, 1-Methyl-4-pentenyl, 2-Methyl-4-pentenyl, 3-Methyl-4-pentenyl, 4-Methyl-4-pentenyl, 1,1-Dimethyl-2-butenyl, 1,1-Dimethyl-3-butenyl, 1,2-Dimethyl-1-butenyl, 1,2-Dimethyl-2-butenyl, 1,2-Dimethyl-3-butenyl, 1,3-Dimethyl-1-butenyl, 1,3-Dimethyl-2-butenyl, 1,3-Dimethyl-3-butenyl, 2,2-Dimethyl-3-butenyl, 2,3-Dimethyl-1-butenyl, 2,3-Dimethyl-2-butenyl, 2,3-Dimethyl-3-butenyl, 3,3-Dimethyl-1-butenyl, 1-Ethyl-1-butenyl, 1-Ethyl-2-butenyl, 1-Ethyl-3-butenyl, 2-Ethyl-1-butenyl, 2-Ethyl-2-butenyl, 2-Ethyl-3-butenyl, 1,1,2-Trimethyl-2-propenyl, 1-Ethyl-1-methyl-2-propenyl, 1-Ethyl-2-methyl-1-propenyl und 1-Ethyl-2-methyl-2-propenyl, insbesondere 1-Methylethenyl bzw. entsprechendes Alkenyloxy und/oder Halogenalkenyl.

Die 6- und 7-gliedrigen Heterocyclen können neben den vorstehend genannten Substituenten auch Hydroxygruppen tragen.

Bei den Phenyl- und Pyridylresten kommen als Substituenten neben den obengenannten Gruppen auch folgende Reste in Betracht:

Alkenyloxy wie 2-Propenyloxy, 2-Butenyloxy, 3-Butenyloxy, 1-Methyl-2-propenyloxy, 2-Methyl-2-propenyloxy, 2-Pentenyloxy, 3-Pentenyloxy, 4-Pentenyloxy, 1-Methyl-2-butenyloxy, 2-Methyl-2-butenyloxy, 3-Methyl-2-butenyloxy, 1-Methyl-3-butenyloxy, 2-Methyl-3-butenyloxy, 3-Methyl-3-butenyloxy, 1,1-Dimethyl-2-propenyloxy, 1,2-Dimethyl-2-propenyloxy, 1-Ethyl-2-propenyloxy, 2-Hexenyloxy, 3-Hexenyloxy, 4-Hexenyloxy, 5-Hexenyloxy, 1-Methyl-2-pentenyloxy, 2-Methyl-2-pentenyloxy, 3-Methyl-2-pentenyloxy, 4-Methyl-2-pentenyloxy, 1-Methyl-3-pentenyloxy, 2-Methyl-3-pentenyloxy, 3-Methyl-3-pentenyloxy, 4-Methyl-3-pentenyloxy, 1-Methyl-4-pentenyloxy, 2-Methyl-4-pentenyloxy, 3-Methyl-4-pentenyloxy, 4-Methyl-4-pentenyloxy, 1,1-Dimethyl-2-butenyloxy, 1,1-Dimethyl-3-butenyloxy, 1,2-Dimethyl-2-butenyloxy, 1,2-Dimethyl-3-butenyloxy, 1,3-Dimethyl-2-butenyloxy, 1,3-Dimethyl-3-butenyloxy, 2,2-Dimethyl-3-butenyloxy, 2,3-Dimethyl-2-butenyloxy, 2,3-Dimethyl-3-butenyloxy, 1-Ethyl-2-butenyloxy, 1-Ethyl-3-butenyloxy, 2-Ethyl-2-butenyloxy, 2-Ethyl-3-butenyloxy, 1,1,2-Trimethyl-2-propenyloxy, 1-Ethyl-1-methyl-2-propenyloxy und 1-Ethyl-2-methyl-2-propenyloxy, insbesondere 2-Propenyloxy und 2-Butenyloxy;

Alkinyloxy wie 2-Propinyloxy, 2-Butinyloxy, 3-Butinyloxy, 1-Methyl-2-propinyloxy, 2-Pentinyloxy, 3-Pentinyloxy, 4-Pentinyloxy, 1-Methyl-3-butinyloxy, 2-Methyl-3-butinyloxy, 1-Methyl-2-butinyloxy, 1,1-Dimethyl-2-propinyloxy, 1-Ethyl-2-propinyloxy, 2-Hexinyloxy, 3-Hexinyloxy, 4-Hexinyloxy, 5-Hexinyloxy, 1-Methyl-2-pentinyloxy, 1-Methyl-3-pentinyloxy, 1-Methyl-4-pentinyloxy, 2-Methyl-3-pentinyloxy, 2-Methyl-4-pentinyloxy, 3-Methyl-4-pentinyloxy, 4-Methyl-2-pentinyloxy, 1,1-Dimethyl-2-butinyloxy, 1,1-Dimethyl-3-butinyloxy, 1,2-Dimethyl-3-butinyloxy, 2,2-Dimethyl-3-butinyloxy, 1-Ethyl-2-butinyloxy, 1-Ethyl-3-butinyloxy, 2-Ethyl-3-butinyloxy und 1-Ethyl-1-methyl-2-propinyloxy, insbesondere 2-Propinyloxy und 2-Butinyloxy;

durch $C_1$-$C_4$-Alkoxy, wie vorstehend im allgemeinen und im besonderen bei X genannt, substituiertes $C_1$-$C_4$-Alkyl wie im allgemeinen und im besonderen vorstehend bei X genannt, vorzugsweise Methoxymethyl, Ethoxymethyl, 1-Methoxyethyl, 2-Methoxyethyl, 1-Ethoxyethyl und 2-Ethoxyethyl;

Amino, welches ein oder zwei der folgenden Reste tragen kann: Alkyl wie bei X genannt, insbesondere Methyl und Ethyl; Alkenyl wie vorstehend genannt, insbesondere 2-Propenyl und 2-Butenyl;

Alkinyl wie Ethinyl, 1-Propinyl, 2-Propinyl, 1-Butinyl, 2-Butinyl, 3-Butinyl, 1-Methyl-2-propinyl, 1-Pentinyl, 2-Pentinyl, 3-Pentinyl, 4-Pentinyl, 1-Methyl-3-butinyl, 2-Methyl-3-butinyl, 1-Methyl-2-butinyl, 3-Methyl-1-butinyl, 1,1-Dimethyl-2-propinyl, 1-Ethyl-2-propinyl, 1-Hexinyl, 2-Hexinyl, 3-Hexinyl, 4-Alkinyl, 5-Hexinyl, 1-Methyl-2-pentinyl, 1-Methyl-3-pentinyl, 1-Methyl-4-pentinyl, 2-Methyl-3-pentinyl, 2-Methyl-4-pentinyl, 3-Methyl-1-pentinyl, 3-Methyl-4-pentinyl, 4-Methyl-1-pentinyl, 4-Methyl-2-pentinyl, 1,1-Dimethyl-2-butinyl, 1,1-Dimethyl-3-butinyl, 1,2-Dimethyl-3-butinyl, 2,2-Dimethyl-3-butinyl, 3,3-Dimethyl-1-butinyl, 1-Ethyl-2-butinyl, 1-Ethyl-3-butinyl, 2-Ethyl-3-butinyl und 1-Ethyl-1-methyl-2-propinyl, insbesondere 2-Propinyl und 2-Butinyl und/oder

Acyl wie Acetyl, Propionyl, Butyryl, 2-Methyl-propionyl, Pentanoyl, 2-Methylbutyryl, 3-Methylbutyryl, 2,2-Dimethylpropionyl, Hexanoyl, 2-Methylpentanoyl, 3-Methylpentanoyl, 4-Methylpentanoyl, 2,2-Dimethylbutyryl, 2,3-Dimethylbutyryl, 3,3-Dimethylbutyryl und 2-Ethylbutyryl, insbesondere Acetyl und Propionyl oder Benzoyl.

Insbesondere bevorzugte Cyclohexenonoximether der Formel I sind in den folgenden Tabellen zusammengefaßt.

Tabelle A

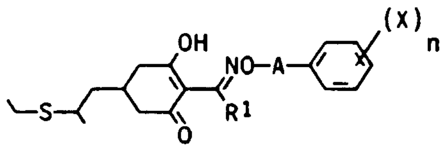

| $R^1$ | A | X | n |
|---|---|---|---|
| $CH_2CH_3$ | $CH_2-C{\equiv}C$ | - | 0 |
| $(CH_2)_2CH_3$ | $CH_2-CH_2-C{\equiv}C$ | - | 0 |
| $CH_2CH_3$ | $CH_2-C{\equiv}C-CH_2$ | - | 0 |
| $(CH_2)_2CH_3$ | $CH_2-CH_2-CH_2-C{\equiv}C$ | - | 0 |
| $CH_2CH_3$ | $CH_2-CH_2-C{\equiv}C-CH_2$ | $4-CF_3$ | 1 |
| $(CH_2)_2CH_3$ | $CH_2-C{\equiv}C-CH_2-CH_2$ | $4-CF_3$ | 1 |
| $CH_2CH_3$ | $CH_2-CH_2-CH_2-C{\equiv}C-CH_2$ | 3-F | 1 |
| $(CH_2)_2CH_3$ | $CH_2-CH_2-C{\equiv}C-CH_2-CH_2$ | 3-F | 1 |
| $CH_2CH_3$ | $CH_2-C{\equiv}C-CH_2-CH_2-CH_2$ | 4-F | 1 |
| $(CH_2)_2CH_3$ | $CH_2-C{\equiv}C$ | 4-F | 1 |
| $CH_2CH_3$ | $CH_2-CH_2-C{\equiv}C$ | $3-CH_3$ | 1 |
| $(CH_2)_2CH_3$ | $CH_2-C{\equiv}C-CH_2$ | $3-CH_3$ | 1 |
| $CH_2CH_3$ | $CH_2-CH_2-CH_2-C{\equiv}C$ | $4-OCH_3$ | 1 |
| $(CH_2)_2CH_3$ | $CH_2-CH_2-C{\equiv}C-CH_2$ | $4-OCH_3$ | 1 |
| $CH_2CH_3$ | $CH_2-C{\equiv}C-CH_2-CH_2$ | $3-NO_2$ | 1 |
| $(CH_2)_2CH_3$ | $CH_2-CH_2-CH_2-C{\equiv}C-CH_2$ | $3-NO_2$ | 1 |
| $CH_2CH_3$ | $CH_2-CH_2-C{\equiv}C-CH_2-CH_2$ | 3-CN | 1 |
| $(CH_2)_2CH_3$ | $CH_2-C{\equiv}C-CH_2-CH_2-CH_2$ | 3-CN | 1 |
| $CH_2CH_3$ | $CH_2-C{\equiv}C$ | $3-CO_2CH_3$ | 1 |
| $(CH_2)_2CH_3$ | $CH_2-CH_2-C{\equiv}C$ | $3-CO_2CH_3$ | 1 |
| $CH_2CH_3$ | $CH_2-C{\equiv}C-CH_2$ | $3-CO_2Ph$ | 1 |
| $(CH_2)_2CH_3$ | $CH_2-CH_2-CH_2-C{\equiv}C$ | $3-CO_2Ph$ | 1 |
| $CH_2CH_3$ | $CH_2-CH_2-C{\equiv}C-CH_2$ | $4-OCHF_2$ | 1 |
| $(CH_2)_2CH_3$ | $CH_2-C{\equiv}C-CH_2-CH_2$ | $4-OCHF_2$ | 1 |
| $CH_2CH_3$ | $CH_2-CH_2-CH_2-C{\equiv}C-CH_2$ | $3-CH_3, 4-Cl$ | 2 |
| $(CH_2)_2CH_3$ | $CH_2-CH_2-C{\equiv}C-CH_2-CH_2$ | $3-CH_3, 4-Cl$ | 2 |

Tabelle B

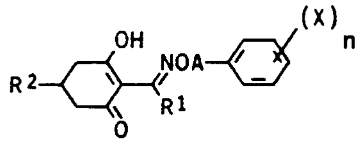

| $R^1$ | $R^2$ | A | X | n |
|---|---|---|---|---|
| $CH_2CH_3$ | 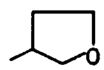 | $CH_2-C\equiv C$ | - | 0 |
| $(CH_2)_2CH_3$ | 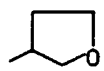 | $CH_2-C\equiv C-CH_2$ | - | 0 |
| $CH_2CH_3$ | 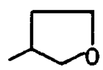 | $CH_2-CH_2-C\equiv C$ | 4-F | 1 |
| $(CH_2)_2CH_3$ | 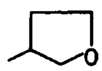 | $CH_2-CH_2-CH_2-C\equiv C$ | 4-F | 1 |
| $CH_2CH_3$ | 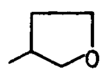 | $CH_2-CH_2-C\equiv C-CH_2$ | $3-CH_3$ | 1 |
| $(CH_2)_2CH_3$ | 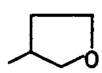 | $CH-C\equiv C-CH_2-CH_2$ | $3-CH_3$ | 1 |
| $CH_2CH_3$ | 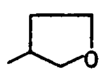 | $CH_2-CH_2-CH_2-CH_2-C\equiv C$ | $3-CF_3$ | 1 |
| $(CH_2)_2CH_3$ | 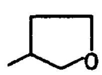 | $CH_2-CH_2-CH_2-C\equiv C-CH_2$ | $3-CF_3$ | 1 |
| $CH_2CH_3$ | 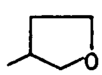 | $CH_2-CH_2-C\equiv C-CH_2-CH_2$ | $4-C(CH_3)_3$ | 1 |
| $(CH_2)_2CH_3$ | 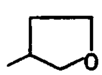 | $CH_2-C\equiv C-CH_2-CH_2-CH_2$ | $4-C(CH_3)_3$ | 1 |
| $CH_2CH_3$ | 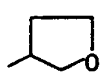 | $CH_2-C\equiv C$ | 3-Cl | 1 |
| $(CH_2)_2CH_3$ | 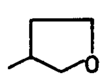 | $CH_2-CH_2-C\equiv C$ | 3-Cl | 1 |

Tabelle B (Fortsetzung)

| $R^1$ | $R^2$ | A | X | n |
|---|---|---|---|---|
| $CH_2CH_3$ | 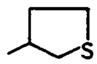 | $CH_2{-}C{\equiv}C{-}CH_2$ | - | 0 |
| $(CH_2)_2CH_3$ | 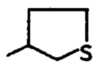 | $CH_2{-}CH_2{-}CH_2{-}C{\equiv}C$ | - | 0 |
| $CH_2CH_3$ | 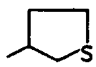 | $CH_2{-}CH_2{-}C{\equiv}C{-}CH_2$ | 4-F | 1 |
| $(CH_2)_2CH_3$ | 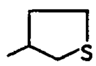 | $CH_2{-}C{\equiv}C{-}CH_2{-}CH_2$ | 4-F | 1 |
| $CH_2CH_3$ | 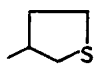 | $CH_2{-}CH_2{-}CH_2{-}CH_2{-}C{\equiv}C$ | 3-$CH_3$ | 1 |
| $(CH_2)_2CH_3$ | 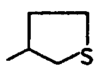 | $CH_2{-}CH_2{-}CH_2{-}C{\equiv}C{-}CH_2$ | 3-$CH_3$ | 1 |
| $CH_2CH_3$ | 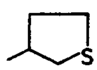 | $CH_2{-}CH_2{-}C{\equiv}C{-}CH_2{-}CH_2$ | 3-$CF_3$ | 1 |
| $(CH_2)_2CH_3$ | 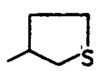 | $CH_2{-}C{\equiv}C{-}CH_2{-}CH_2{-}CH_2$ | 3-$CF_3$ | 1 |
| $CH_2CH_3$ | 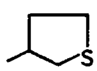 | $CH_2{-}C{\equiv}C$ | 4-$C(CH_3)_3$ | 1 |
| $(CH_2)_2CH_3$ | 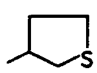 | $CH_2{-}CH_2{-}C{\equiv}C$ | 4-$C(CH_3)_3$ | 1 |
| $CH_2CH_3$ | 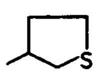 | $CH_2{-}C{\equiv}C{-}CH_2$ | 3-Cl | 1 |
| $(CH_2)_2CH_3$ | 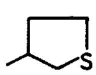 | $CH_2{-}CH_2{-}CH_2{-}C{\equiv}C$ | 3-Cl | 1 |
| $CH_2CH_3$ | 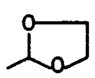 | $CH_2{-}CH_2{-}C{\equiv}C{-}CH_2$ | - | 0 |
| $(CH_2)_2CH_3$ | 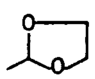 | $CH_2{-}C{\equiv}C{-}CH_2{-}CH_2$ | - | 0 |

Tabelle B (Fortsetzung)

| $R^1$ | $R^2$ | A | X | n |
|---|---|---|---|---|
| $CH_2CH_3$ | 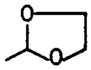 | $CH_2-CH_2-CH_2-CH_2-C{\equiv}C$ | 4-F | 1 |
| $(CH_2)_2CH_3$ | 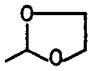 | $CH_2-CH_2-CH_2-C{\equiv}C-CH_2$ | 4-F | 1 |
| $CH_2CH_3$ | 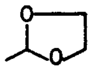 | $CH_2-CH_2-C{\equiv}C-CH_2-CH_2$ | $3-CH_3$ | 1 |
| $(CH_2)_2CH_3$ | 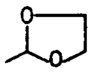 | $CH_2-C{\equiv}C-CH_2-CH_2-CH_2$ | $3-CH_3$ | 1 |
| $CH_2CH_3$ | 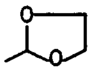 | $CH_2-C{\equiv}C$ | $3-CF_3$ | 1 |
| $(CH_2)_2CH_3$ | 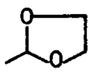 | $CH_2-CH_2-C{\equiv}C$ | $3-CF_3$ | 1 |
| $CH_2CH_3$ | 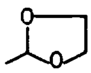 | $CH_2-C{\equiv}C-CH_2$ | $4-C(CH_3)_3$ | 1 |
| $(CH_2)_2CH_3$ | 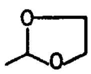 | $CH_2-CH_2-CH_2-C{\equiv}C$ | $4-C(CH_3)_3$ | 1 |
| $CH_2CH_3$ | 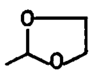 | $CH_2-CH_2-C{\equiv}C-CH_2$ | 3-Cl | 1 |
| $(CH_2)_2CH_3$ | 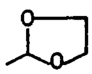 | $CH_2-C{\equiv}C-CH_2-CH_2$ | 3-Cl | 1 |
| $CH_2CH_3$ | 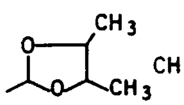 | $CH_2-CH_2-CH_2-CH_2-C{\equiv}C$ | - | 0 |
| $(CH_2)_2CH_3$ | 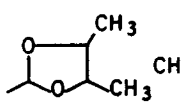 | $CH_2-CH_2-CH_2-C{\equiv}C-CH_2$ | - | 0 |
| $CH_2CH_3$ | 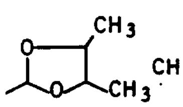 | $CH_2-CH_2-C{\equiv}C-CH_2-CH_2$ | 4-F | 1 |

Tabelle B (Fortsetzung)

| $R^1$ | $R^2$ | A | X | n |
|---|---|---|---|---|
| $(CH_2)_2CH_3$ | 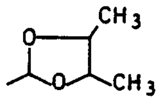 | $CH_2{-}C{\equiv}C{-}CH_2{-}CH_2{-}CH_2$ | 4-F | 1 |
| $CH_2CH_3$ | 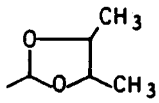 | $CH_2{-}C{\equiv}C$ | $3{-}CH_3$ | 1 |
| $(CH_2)_2CH_3$ | 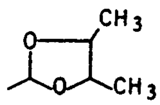 | $CH_2{-}CH_2{-}C{\equiv}C$ | $3{-}CH_3$ | 1 |
| $CH_2CH_3$ | 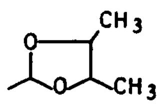 | $CH_2{-}C{\equiv}C{-}CH_2$ | $3{-}CF_3$ | 1 |
| $(CH_2)_2CH_3$ | 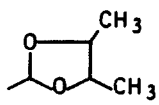 | $CH_2{-}CH_2{-}CH_2{-}C{\equiv}C$ | $3{-}CF_3$ | 1 |
| $CH_2CH_3$ | 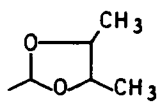 | $CH_2{-}CH_2{-}C{\equiv}C{-}CH_2$ | $4{-}C(CH_3)_3$ | 1 |
| $(CH_2)_2CH_3$ | 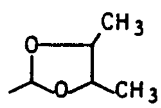 | $CH_2{-}C{\equiv}C{-}CH_2{-}CH_2$ | $4{-}C(CH_3)_3$ | 1 |
| $CH_2CH_3$ | 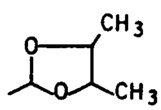 | $CH_2{-}CH_2{-}CH_2{-}CH_2{-}C{\equiv}C$ | 3-Cl | 1 |
| $(CH_2)_2CH_3$ | 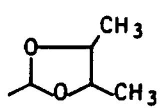 | $CH_2{-}CH_2{-}CH_2{-}C{\equiv}C{-}CH_2$ | 3-Cl | 1 |
| $CH_2CH_3$ | 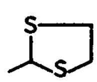 | $CH_2{-}CH_2{-}C{\equiv}C{-}CH_2{-}CH_2$ | - | 0 |
| $(CH_2)_2CH_3$ | 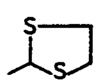 | $CH_2{-}C{\equiv}C{-}CH_2{-}CH_2{-}CH_2$ | - | 0 |
| $CH_2CH_3$ | 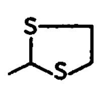 | $CH_2{-}C{\equiv}C$ | 4-F | 1 |

Tabelle B (Fortsetzung)

| $R^1$ | $R^2$ | A | X | n |
|---|---|---|---|---|
| $(CH_2)_2CH_3$ | 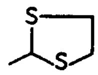 | $CH_2-CH_2-C \equiv C$ | 4-F | 1 |
| $CH_2CH_3$ | 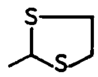 | $CH_2-C \equiv C-CH_2$ | $3-CH_3$ | 1 |
| $(CH_2)_2CH_3$ | 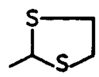 | $CH_2-CH_2-CH_2-C \equiv C$ | $3-CH_3$ | 1 |
| $CH_2CH_3$ | 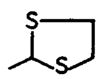 | $CH_2-CH_2-C \equiv C-CH_2$ | $3-CF_3$ | 1 |
| $(CH_2)_2CH_3$ | 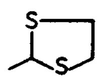 | $CH_2-C \equiv C-CH_2-CH_2$ | $3-CF_3$ | 1 |
| $CH_2CH_3$ | 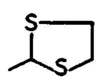 | $CH_2-CH_2-CH_2-CH_2-C \equiv C$ | $4-C(CH_3)_3$ | 1 |
| $(CH_2)_2CH_3$ | 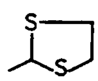 | $CH_2-CH_2-CH_2-C \equiv C-CH_2$ | $4-C(CH_3)_3$ | 1 |
| $CH_2CH_3$ | 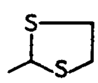 | $CH_2-CH_2-C \equiv C-CH_2-CH_2$ | 3-Cl | 1 |
| $(CH_2)_2CH_3$ | 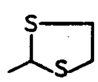 | $CH_2-C \equiv C-CH_2-CH_2-CH_2$ | 3-Cl | 1 |
| $CH_2CH_3$ | 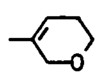 | $CH_2-C \equiv C$ | - | 0 |
| $(CH_2)_2CH_3$ | 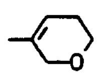 | $CH_2-CH_2-C \equiv C$ | - | 0 |
| $CH_2CH_3$ | 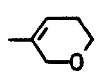 | $CH_2-C \equiv C-CH_2$ | 4-F | 1 |
| $(CH_2)_2CH_3$ | 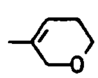 | $CH_2-CH_2-CH_2-C \equiv C$ | 4-F | 1 |

Tabelle B (Fortsetzung)

| $R^1$ | $R^2$ | A | X | n |
|---|---|---|---|---|
| $CH_2CH_3$ | 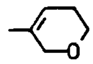 | $CH_2-CH_2-C{\equiv}C-CH_2$ | $3-CH_3$ | 1 |
| $(CH_2)_2CH_3$ | 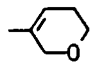 | $CH_2-C{\equiv}C-CH_2-CH_2$ | $3-CH_3$ | 1 |
| $CH_2CH_3$ | 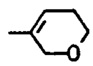 | $CH_2-CH_2-CH_2-CH_2-C{\equiv}C$ | $3-CF_3$ | 1 |
| $(CH_2)_2CH_3$ | 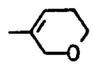 | $CH_2-CH_2-CH_2-C{\equiv}C-CH_2$ | $3-CF_3$ | 1 |
| $CH_2CH_3$ | 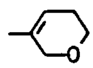 | $CH_2-CH_2-C{\equiv}C-CH_2-CH_2$ | $4-C(CH_3)_3$ | 1 |
| $(CH_2)_2CH_3$ | 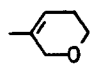 | $CH_2-C{\equiv}C-CH_2-CH_2-CH_2$ | $4-C(CH_3)_3$ | 1 |
| $CH_2CH_3$ | 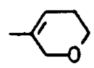 | $CH_2-C{\equiv}C$ | 3-Cl | 1 |
| $(CH_2)_2CH_3$ | 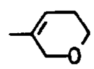 | $CH_2-CH_2-C{\equiv}C$ | 3-Cl | 1 |
| $CH_2CH_3$ | 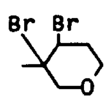 | $CH_2-C{\equiv}C-CH_2$ | - | 0 |
| $(CH_2)_2CH_3$ | 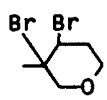 | $CH_2-CH_2-CH_2-C{\equiv}C$ | - | 0 |
| $CH_2CH_3$ | 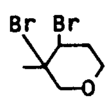 | $CH_2-CH_2-C{\equiv}C-CH_2$ | 4-F | 1 |
| $(CH_2)_2CH_3$ | 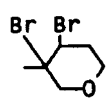 | $CH_2-C{\equiv}C-CH_2-CH_2$ | 4-F | 1 |
| $CH_2CH_3$ | 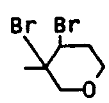 | $CH_2-CH_2-CH_2-CH_2-C{\equiv}C$ | $3-CH_3$ | 1 |

Tabelle B (Fortsetzung)

| $R^1$ | $R^2$ | A | X | n |
|---|---|---|---|---|
| $(CH_2)_2CH_3$ | 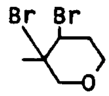 | $CH_2-CH_2-CH_2-C\equiv C-CH_2$ | $3-CH_3$ | 1 |
| $CH_2CH_3$ | 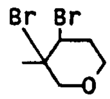 | $CH_2-CH_2-C\equiv C-CH_2-CH_2$ | $3-CF_3$ | 1 |
| $(CH_2)_2CH_3$ | 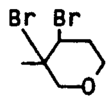 | $CH_2-C\equiv C-CH_2-CH_2-CH_2$ | $3-CF_3$ | 1 |
| $CH_2CH_3$ | 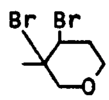 | $CH_2-C\equiv C$ | $4-C(CH_3)_3$ | 1 |
| $(CH_2)_2CH_3$ | 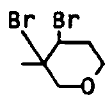 | $CH_2-CH_2-C\equiv C$ | $4-C(CH_3)_3$ | 1 |
| $CH_2CH_3$ | 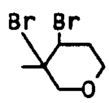 | $CH_2-C\equiv C-CH_2$ | 3-Cl | 1 |
| $(CH_2)_2CH_3$ | 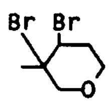 | $CH_2-CH_2-CH_2-C\equiv C$ | 3-Cl | 1 |
| $CH_2CH_3$ | 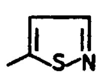 | $CH_2-CH_2-C\equiv C-CH_2$ | - | 0 |
| $(CH_2)_2CH_3$ | 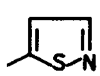 | $CH_2-C\equiv C-CH_2-CH_2$ | - | 0 |
| $CH_2CH_3$ | 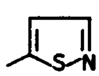 | $CH_2-CH_2-CH_2-CH_2-C\equiv C$ | 4-F | 1 |
| $(CH_2)_2CH_3$ | 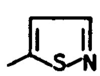 | $CH_2-CH_2-CH_2-C\equiv C-CH_2$ | 4-F | 1 |
| $CH_2CH_3$ | 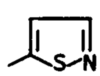 | $CH_2-CH_2-C\equiv C-CH_2-CH_2$ | $3-CH_3$ | 1 |

Tabelle B (Fortsetzung)

| $R^1$ | $R^2$ | A | X | n |
|---|---|---|---|---|
| $(CH_2)_2CH_3$ | 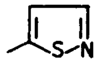 | $CH_2-C{\equiv}C-CH_2-CH_2-CH_2$ | $3-CH_3$ | 1 |
| $CH_2CH_3$ | 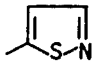 | $CH_2-C{\equiv}C$ | $3-CF_3$ | 1 |
| $(CH_2)_2CH_3$ | 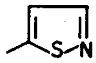 | $CH_2-CH_2-C{\equiv}C$ | $3-CF_3$ | 1 |
| $CH_2CH_3$ | 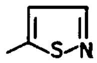 | $CH_2-C{\equiv}C-CH_2$ | $4-C(CH_3)_3$ | 1 |
| $(CH_2)_2CH_3$ | 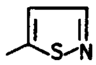 | $CH_2-CH_2-CH_2-C{\equiv}C$ | $4-C(CH_3)_3$ | 1 |
| $CH_2CH_3$ | 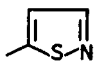 | $CH_2-CH_2-C{\equiv}C-CH_2$ | 3-Cl | 1 |
| $(CH_2)_2CH_3$ | 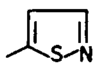 | $CH_2-C{\equiv}C-CH_2-CH_2$ | 3-Cl | 1 |
| $CH_2CH_3$ | 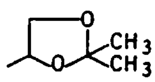 | $CH_2-CH_2-CH_2-CH_2-C{\equiv}C$ | $3-CF_3$ | 1 |
| $(CH_2)_2CH_3$ | 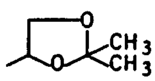 | $CH_2-CH_2-CH_2-C{\equiv}C-CH_2$ | $3-CF_3$ | 1 |
| $CH_2CH_3$ | 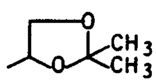 | $CH_2-CH_2-C{\equiv}C-CH_2-CH_2$ | $4-C(CH_3)_3$ | 1 |
| $(CH_2)_2CH_3$ | 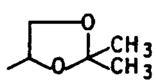 | $CH_2-C{\equiv}C-CH_2-CH_2-CH_2$ | $4-C(CH_3)_3$ | 1 |
| $CH_2CH_3$ | 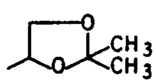 | $CH_2-C{\equiv}C$ | - | 0 |
| $(CH_2)_2CH_3$ | 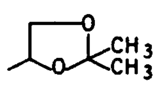 | $CH_2-CH_2-C{\equiv}C$ | - | 0 |
| $CH_2CH_3$ | 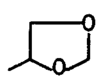 | $CH_2-C{\equiv}C-CH_2$ | - | 0 |

Tabelle B (Fortsetzung)

| $R^1$ | $R^2$ | X | n |
|---|---|---|---|
| $(CH_2)_2CH_3$ | 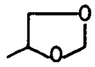 $CH_2-C{\equiv}C-CH_2$ | - | 0 |
| $CH_2CH_3$ | 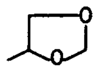 $CH_2-CH_2-CH_2-C{\equiv}C$ | $3-CF_3$ | 1 |
| $(CH_2)_2CH_3$ | 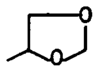 $CH_2-CH_2-C{\equiv}C-CH_2$ | $3-CF_3$ | 1 |
| $CH_2CH_3$ | 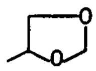 $CH_2-C{\equiv}C-CH_2-CH_2$ | $4-C(CH_3)_3$ | 1 |
| $(CH_2)_2CH_3$ | 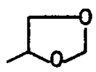 $CH_2-CH_2-CH_2-CH_2-C{\equiv}C$ | $4-C(CH_3)_3$ | 1 |
| $CH_2CH_3$ | 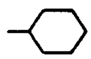 $CH_2-CH_2-CH_2-C{\equiv}C-CH_2$ | - | 0 |
| $(CH_2)_2CH_3$ | 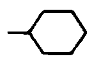 $CH_2-CH_2-C{\equiv}C-CH_2-CH_2$ | - | 0 |
| $CH_2CH_3$ | 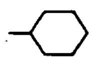 $CH_2-C{\equiv}C-CH_2-CH_2-CH_2$ | $3-CF_3$ | 1 |
| $(CH_2)_2CH_3$ | 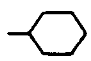 $CH_2-C{\equiv}C$ | $3-CF_3$ | 1 |
| $CH_2CH_3$ | 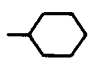 $CH_2-CH_2-C{\equiv}C$ | $4-C(CH_3)_3$ | 1 |
| $(CH_2)_2CH_3$ | 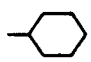 $CH_2-C{\equiv}C-CH_2$ | $4-C(CH_3)_3$ | 1 |
| $CH_2CH_3$ | 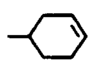 $CH_2-CH_2-CH_2-C{\equiv}C$ | - | 0 |
| $(CH_2)_2CH_3$ | 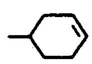 $CH_2-CH_2-C{\equiv}C$ | - | 0 |
| $CH_2CH_3$ | 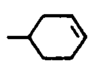 $CH_2-C{\equiv}C-CH_2$ | $3-CF_3$ | 1 |
| $(CH_2)_2CH_3$ | 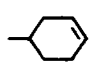 $CH_2-C{\equiv}C$ | $3-CF_3$ | 1 |
| $CH_2CH_3$ | 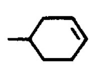 $CH_2-C{\equiv}C-CH_2$ | $4-C(CH_3)_3$ | 1 |
| $(CH_2)_2CH_3$ | 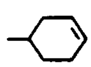 $CH_2-CH_2-C{\equiv}C$ | $4-C(CH_3)_3$ | 1 |

Tabelle B (Fortsetzung)

| $R^1$ | $R^2$ | A | X | n |
|---|---|---|---|---|
| $(CH_2)_2CH_3$ | 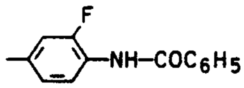 | $CH_2-C\equiv C-CH_2$ | - | 0 |
| $CH_2CH_3$ | 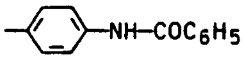 | $CH_2-CH_2-CH_2-C\equiv C$ | $3-CF_3$ | 1 |
| $(CH_2)_2CH_3$ | 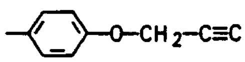 | $CH_2-CH_2-C\equiv C-CH_2$ | $3-CF_3$ | 1 |
| $CH_2CH_3$ | 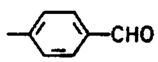 | $CH_2-C\equiv C-CH_2-CH_2$ | $4-C(CH_3)_3$ | 1 |
| $(CH_2)_2CH_3$ | 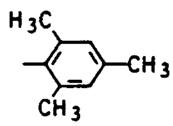 | $CH_2-CH_2-CH_2-CH_2-C\equiv C$ | $4-C(CH_3)_3$ | 1 |
| $CH_2CH_3$ | 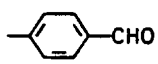 | $CH_2-CH_2-CH_2-C\equiv C-CH_2$ | - | 0 |
| $(CH_2)_2CH_3$ | 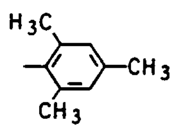 | $CH_2-CH_2-C\equiv C-CH_2-CH_2$ | - | 0 |
| $CH_2CH_3$ | 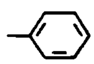 | $CH_2-C\equiv C-CH_2-CH_2-CH_2$ | $3-CF_3$ | 1 |
| $(CH_2)_2CH_3$ | 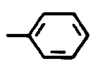 | $CH_2-C\equiv C$ | $3-CF_3$ | 1 |
| $CH_2CH_3$ | 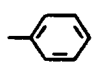 | $CH_2-CH_2-C\equiv C$ | $4-C(CH_3)_3$ | 1 |
| $(CH_2)_2CH_3$ | 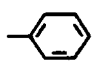 | $CH_2-C\equiv C-CH_2$ | $4-C(CH_3)_3$ | 1 |
| $CH_2CH_3$ | 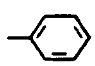 | $CH_2-CH_2-CH_2-C\equiv C$ | - | 0 |
| $(CH_2)_2CH_3$ | 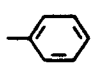 | $CH_2-CH_2-C\equiv C$ | - | 0 |
| $CH_2CH_3$ | 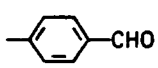 | $CH_2-C\equiv C-CH_2$ | $3-CF_3$ | 1 |
| $(CH_2)_2CH_3$ | 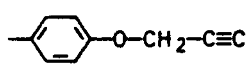 | $CH_2-C\equiv C$ | $3-CF_3$ | 1 |

Tabelle B (Fortsetzung)

| $R^1$ | $R^2$ | A | X | n |
|---|---|---|---|---|
| $CH_2CH_3$ | 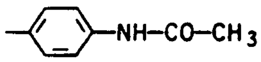 | $CH_2-C\equiv C-CH_2$ | $4-C(CH_3)_3$ | 1 |
| $(CH_2)_2CH_3$ | 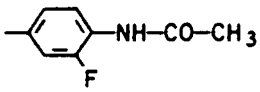 | $CH_2-CH_2-C\equiv C$ | $4-C(CH_3)_3$ | 1 |

Die Cyclohexenonoximether I eignen sich als Herbizide, insbesondere zur Bekämpfung von Pflanzenarten aus der Familie der Gramineen (Gräser).

Die Cyclohexenonoximether I bzw. die sie enthaltenden herbiziden Mittel können beispielsweise in Form von direkt versprühbaren Lösungen, Pulvern, Suspensionen, auch hochprozentigen wäßrigen, öligen oder sonstigen Suspensionen oder Dispersionen, Emulsionen, Öldispersionen, Pasten, Stäubemitteln, Streumitteln oder Granulaten durch Versprühen, Vernebeln, Verstäuben, Verstreuen oder Gießen angewendet werden. Die Anwendungsformen richten sich nach den Verwendungszwecken; sie sollten in jedem Fall möglichst die feinste Verteilung der erfindungsgemäßen Wirkstoffe gewährleisten.

Die Verbindungen I eignen sich allgemein zur Herstellung von direkt versprühbaren Lösungen, Emulsionen, Pasten oder Öldispersionen. Als inerte Zusatzstoffe kommen Mineralölfraktionen von mittlerem bis hohem Siedepunkt, wie Kerosin oder Dieselöl, ferner Kohlenteeröle sowie Öle pflanzlichen oder tierischen Ursprungs, aliphatische, cyclische und aromatische Kohlenwasserstoffe, z.B. Toluol, Xylol, Paraffin, Tetrahydronaphthalin, alkylierte Naphthaline oder deren Derivate, Methanol, Ethanol, Propanol, Butanol, Cyclohexanol, Cyclohexanon, Chlorbenzol, Isophoron oder stark polare Lösungsmittel, wie N,N-Dimethylformamid, Dimethylsulfoxid, N-Methylpyrrolidon oder Wasser in Betracht.

Wäßrige Anwendungsformen können aus Emulsionskonzentraten, Dispersionen, Pasten, netzbaren Pulvern oder wasserdispergierbaren Granulaten durch Zusatz von Wasser bereitet werden. Zur Herstellung von Emulsionen, Pasten oder Öldispersionen können die Substrate als solche oder in einem Öl oder Lösungsmittel gelöst, mittels Netz-, Haft-, Dispergier- oder Emulgiermittel in Wasser homogenisiert werden. Es können aber auch aus wirksamer Substanz, Netz-, Haft-, Dispergier- oder Emulgiermittel und eventuell Lösungsmittel oder Öl bestehende Konzentrate hergestellt werden, die zur Verdünnung mit Wasser geeignet sind.

Als oberflächenaktive Stoffe kommen die Alkali-, Erdalkali-, Ammoniumsalze von aromatischen Sulfonsäuren, z.B. Lignin-, Phenol-, Naphthalin-und Dibutylnaphthalinsulfonsäure, sowie von Fettsäuren, Alkyl- und Alkylarylsulfonaten, Alkyl-, Laurylether- und Fettalkoholsulfaten, sowie Salze sulfatierter Hexa-, Hepta- und Octadecanolen, sowie von Fettalkoholglykolether, Kondensationsprodukte von sulfoniertem Naphthalin und seiner Derivate mit Formaldehyd, Kondensationsprodukte des Naphthalins bzw. der Naphthalinsulfonsäuren mit Phenol und Formaldehyd, Polyoxyethylenoctylphenolether, ethoxyliertes Isooctyl-, Octyl- oder Nonylphenol, Alkylphenol-, Tributylphenylpolyglykolether, Alkylarylpolyetheralkohole, Isotridecylalkohol, Fettalkoholethylenoxid-Kondensate, ethoxyliertes Rizinusöl, Polyoxyethylenalkylether oder Polyoxypropylen, Laurylalkoholpolyglykoletheracetat, Sorbitester, Lignin-Sulfitablaugen oder Methylcellulose in Betracht.

Pulver-, Streu- und Stäubemittel können durch Mischen oder gemeinsames Vermahlen der wirksamen Substanzen mit einem festen Trägerstoff hergestellt werden.

Granulate, z.B. umhüllungs-, Imprägnierungs- und Homogengranulate können durch Bindung der Wirkstoffe an feste Trägerstoffe hergestellt werden. Feste Trägerstoffe sind Mineralerden wie Kieselsäuren, Kieselgele, Silikate, Talkum, Kaolin, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie Ammoniumsulfat, Ammoniumphosphat, Ammoniumnitrat, Harnstoffe und pflanzliche Produkte, wie Getreidemehl, Baumrinden-, Holz- und Nußschalenmehl, Cellulosepulver oder andere feste Trägerstoffe.

Die Formulierungen enthalten zwischen 0,02 und 95 Gew.%, vorzugsweise zwischen 0,5 und 90 Gew.%, Wirkstoff. Die Wirkstoffe werden dabei in einer Reinheit von 90 % bis 100 %, vorzugsweise 95 % bis 100 % (nach NMR-Spektrum) eingesetzt.

Die erfindungsgemäßen Verbindungen I können beispielsweise wie folgt formuliert werden:

I. Man vermischt 90 Gewichtsteile der Verbindung Nr. 3.2 mit 10 Gewichtsteilen N-Methyl-α-pyrrolidon und erhält eine Lösung, die zur Anwendung in Form kleinster Tropfen geeignet ist.

II. 20 Gewichtsteile der Verbindung Nr. 3.4 werden in einer Mischung gelöst, die aus 80 Gewichtsteilen Xylol, 10 Gewichtsteilen des Anlagerungsproduktes von 8 bis 10 Mol Ethylenoxid an 1 Mol Ölsäure-N-monoethanolamid, 5 Gewichtsteilen Calciumsalz der Dodecylbenzolsulfonsäure und 5 Gewichtsteilen des Anlagerungsproduktes von 40 Mol Ethylenoxid an 1 Mol Ricinusöl besteht. Durch Ausgießen und feines Verteilen der Lösung in 100 000 Gewichtsteilen Wasser erhält man eine wäßrige Dispersion, die 0,02 Gew.% des Wirkstoffs enthält.

III. 20 Gewichtsteile der Verbindung Nr. 3.4 werden in einer Mischung gelöst, die aus 40 Gewichtsteilen Cyclohexanon, 30 Gewichtsteilen Isobutanol, 20 Gewichtsteilen des Anlagerungsproduktes von 7 Mol Ethylenoxid an 1 Mol Isooctylphenol und 10 Gewichtsteilen des Anlagerungsproduktes von 40 Mol Ethylenoxid an 1 Mol Ricinusöl besteht. Durch Eingießen und feines Verteilen der Lösung in 100 000 Gewichtsteilen Wasser erhält man eine wäßrige Dispersion, die 0,02 Gew.% des Wirkstoffs enthält.

IV. 20 Gewichtsteile des Wirkstoffs Nr. 3.2 werden in einer Mischung gelöst, die aus 25 Gewichtsteilen Cyclohexanon, 65 Gewichtsteilen einer Mineralölfraktion vom Siedepunkt 210 bis 280 °C und 10 Gewichtsteilen des Anlagerungsproduktes von 40 Mol Ethylenoxid an 1 Mol Ricinusöl besteht. Durch Eingießen und feines Verteilen der Lösung in 100 000 Gewichtsteilen Wasser erhält man eine wäßrige Dispersion, die 0,02 Gew.% des Wirkstoffs enthält.

V. 20 Gewichtsteile des Wirkstoffs Nr. 3.4 werden mit 3 Gewichtsteilen des Natriumsalzes der Diisobutylnaphthalin-$\alpha$-sulfonsäure, 17 Gewichtsteilen des Natriumsalzes einer Ligninsulfonsäure aus einer Sulfit-Ablauge und 60 Gewichtsteilen pulverförmigem Kieselsäuregel gut vermischt und in einer Hammermühle vermahlen. Durch feines Verteilen der Mischung in 20 000 Gewichtsteilen Wasser erhält man eine Spritzbrühe, die 0,1 Gew.% des Wirkstoffs enthält.

VI. 3 Gewichtsteile des Wirkstoffs Nr. 3.2 werden mit 97 Gewichtsteilen feinteiligem Kaolin vermischt. Man erhält auf diese Weise ein Stäubemittel, das 3 Gew.% des Wirkstoffs enthält.

VII. 30 Gewichtsteile des Wirkstoffs Nr. 3.4 werden mit einer Mischung aus 92 Gewichtsteilen pulverförmigem Kieselsäuregel und 8 Gewichtsteilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit.

VIII. 20 Gewichtsteile des Wirkstoffs Nr. 3.2 werden mit 2 Gewichtsteilen Calciumsalz der Dodecylbenzolsulfonsäure, 8 Gewichtsteilen Fettalkohol-polyglykolether, 2 Gewichtsteilen Natriumsalz eines Phenol-Harnstoff-Formaldehyd-Kondensates und 68 Gewichtsteilen eines paraffinischen Mineralöls innig vermischt. Man erhält eine stabile ölige Dispersion.

Die Applikation der Mittel kann im Vorauflauf- oder im Nachauflaufverfahren erfolgen. Sind die Wirkstoffe für gewisse Kulturpflanzen weniger verträglich, so können auch Ausbringungstechniken angewandt werden, bei welchen die herbiziden Mittel mit Hilfe der Spritzgeräte so gespritzt werden, daß die Blätter der empfindlichen Kulturpflanzen nach Möglichkeit nicht getroffen werden, während die Wirkstoffe auf die Blätter darunter wachsender unerwünschter Pflanzen oder die unbedeckte Bodenfläche gelangen (post-directed, lay-by).

Die Aufwandmengen an Wirkstoff betragen je nach Jahreszeit, Zielpflanzen und Wachstumsstadium 0,001 bis 3 kg/ha, vorzugsweise 0,01 bis 2 kg/ha.

In Anbetracht des erfaßbaren Wirkungsspektrums zur Unkrautbekämpfung, der Verträglichkeit für Kulturpflanzen oder der erwünschten Beeinflussung des Wachstums derselben sowie angesichts der Vielfalt der Applikationsmethoden können die erfindungsgemäßen Verbindungen in einer großen Zahl von Kulturpflanzen eingesetzt werden. In Betracht kommen beispielsweise folgende Kulturen:

| Botanischer Name | Deutscher Name |
|---|---|
| Allium cepa | Küchenzwiebel |
| Ananas comosus | Ananas |
| Arachis hypogaea | Erdnuß |
| Asparagus officinalis | Spargel |
| Beta vulgaris spp. altissima | Zuckerrübe |
| Beta vulgaris spp. rapa | Futterrübe |
| Brassica napus var. napus | Raps |
| Brassica napus var. napobrassica | Kohlrübe |
| Brassica rapa var. silvestris | Rübsen |
| Camellia sinensis | Teestrauch |
| Carthamus tinctorius | Saflor - Färberdistel |
| Carya illinoinensis | Pekannußbaum |
| Citrus limon | Zitrone |
| Citrus sinensis | Apfelsine, Orange |
| Coffea arabica (Coffea canephora, Coffea liberica) | Kaffee |
| Cucumis melo | Melone |
| Cucumis sativus | Gurke |

| Botanischer Name | Deutscher Name |
|---|---|
| Cynodon dactylon | Bermudagras |
| Daucus carota | Möhre |
| Elaeis guineensis | Ölpalme |
| Fragaria vesca | Erdbeere |
| Glycine max | Sojabohne |
| Gossypium hirsutum (Gossypium arboreum, Gossypium herbaceum, Gossypium vitifolium) | Baumwolle |
| Helianthus annuus | Sonnenblume |
| Hevea brasiliensis | Parakautschukbaum |
| Hordeum vulgare | Gerste |
| Humulus lupulus | Hopfen |
| Ipomoea batatas | Süßkartoffeln |
| Juglans regia | Walnußbaum |
| Lens culinaris | Linse |
| Linum usitatissimum | Faserlein |
| Lycopersicon lycopersicum | Tomate |
| Malus spp. | Apfel |
| Manihot esculenta | Maniok |
| Medicago sativa | Luzerne |
| Musa spp. | Obst- und Mehlbanane |
| Nicotiana tabacum (N. rustica) | Tabak |
| Olea europaea | Ölbaum |
| Oryza sativa | Reis |
| Phaseolus lunatus | Mondbohne |
| Phaseolus vulgaris | Buschbohnen |
| Picea abies | Rotfichte |
| Pinus spp. | Kiefer |
| Pisum sativum | Gartenerbse |
| Prunus avium | Süßkirsche |
| Prunus persica | Pfirsich |
| Pyrus communis | Birne |
| Ribes sylvestre | Rote Johannisbeere |
| Ricinus communis | Rizinus |
| Saccharum officinarum | Zuckerrohr |
| Secale cereale | Roggen |
| Solanum tuberosum | Kartoffel |
| Sorghum bicolor (s. vulgare) | Mohrenhirse |
| Theobroma cacao | Kakaobaum |
| Trifolium pratense | Rotklee |
| Triticum aestivum | Weizen |
| Triticum durum | Hartweizen |
| Vicia faba | Pferdebohnen |
| Vitis vinifera | Weinrebe |
| Zea mays | Mais |

Zur Verbreiterung des Wirkungsspektrums und zur Erzielung synergistischer Effekte können die Cyclohexenonderivate der Formel I sowohl untereinander als auch mit Vertretern anderer herbizider oder wachstumsregulierender Wirkstoffgruppen gemischt und gemeinsam ausgebracht werden. Beispielsweise kommen als Mischungspartner Diazine, 4H-3,1-Benzoxazinderivate, Benzothiadiazinone, 2,6-Dinitroaniline, N-Phenylcarbamate, Thiolcarbamate, Halogencarbonsäuren, Triazine, Amide, Harnstoffe, Diphenylether, Triazinone, Uracile, Benzofuranderivate, Chinolincarbonsäuren, Cyclohexenone, (Hetero)-

aryloxyphenoxypropionsäuren, deren Salze, Ester und Amide und andere in Betracht.

Außerdem kann es von Nutzen sein, die Cyclohexenonderivate der Formel I bzw. sie enthaltende herbizide Mittel allein oder in Kombination mit anderen Herbiziden oder auch noch mit weiteren Pflanzenschutzmitteln gemischt gemeinsam auszubringen, beispielsweise mit Mitteln zur Bekämpfung von Schädlingen oder phytopathogenen Pilzen bzw. Bakterien. Von Interesse ist ferner die Mischbarkeit mit Mineralsalzlösungen, welche zur Behebung von Ernährungs- und Spurenelementmängeln eingesetzt werden. Es können auch nichtphytotoxische Öle und Ölkonzentrate zugesetzt werden.

Die im folgenden beschriebenen Synthesen für die neuen Hydroxylamine lassen sich unter entsprechender Abwandlung der Ausgangsverbindung zur Gewinnung weiterer Verbindungen der Formel III benutzen. Die erhaltenen Verbindungen sind in den Tabellen 1 bis 3 zusammengefaßt.

Herstellbeispiele

4-(4-Fluorphenyl)-3-butinol (Bsp. 1.1)

Eine Lösung von 100 g 4-Bromfluorbenzol in 350 ml Triethylamin wurde nacheinander mit 1 g Bis-(triphenylphosphin)-palladium-(II)-chlorid, 3,8 g Kupfer-(I)-jodid und 8,7 g Triphenylphosphin versetzt. Diese Mischung wurde auf Rückflußtemperatur erwärmt, wonach man bei dieser Temperatur (ca. 100 °C) 43,4 g 3-Butinol innerhalb 20 min zutropfte. Es wurde noch 5 h bei dieser Temperatur gerührt. Nach dem Abkühlen wurde das Triethylamin abdestilliert. Der Rückstand wurde in Methyl-tert.-butylether und Wasser aufgenommen. Die wäßrige Phase wurde noch zweimal mit Methyl-tert.-butylether extrahiert, die vereinigten organischen Extrakte wurden nacheinander mit 1 N Salzsäure und mit 10%iger Natriumbicarbonatlösung gewaschen, über Natriumsulfat getrocknet und am Rotationsverdampfer eingeengt. Nach der Destillation im Hochvakuum erhielt man 80 g (86%) der gewünschten Verbindung.

5-Aminooxy-1-(4-fluorphenyl)-1-pentin (Bsp. 2.6)

N-(5-(4-Fluorphenyl)-4-pentinyloxy)phthalimid (Bsp. 1.10)

Zu einer Lösung von 33,1 g (0,186 mol) 5-Hydroxy-1-(4-fluorphenyl)-1-pentin in 430 ml trockenem Tetrahydrofuran wurden 33,4 g (0,205 mol) N-Hydroxyphthalimid und 53,8 g (0,205 mol) Triphenylphosphin gegeben. Innerhalb von 2,5 h tropfte man dann unter Temperaturkontrolle (max. 40 °C) 35,7 g (0,205 mol) Diethylazodicarboxylat zu. Man rührte über Nacht bei Raumtemperatur, engte die Mischung im Vakuum ein und nahm mit 300 ml Dichlormethan auf. Es wurde zweimal mit Natriumcarbonatlösung und einmal mit gesättigter Kochsalzlösung gewaschen. Nach Trocknen und Einengen wurde das Rohprodukt an Kieselgel chromatographisch gereinigt. Als Eluens wurde zunächst Dichlormethan/n-Hexan benutzt, später dann reines Dichlormethan. Ausbeute: 49 g (82%); Fp. 87-88 °C.
250-MHz-$^1$H-NMR(DMSO-d6):
$\delta$ (ppm) = 1,9-2,1(m,2H); 2,68(t,2H); 4,32(t,2H); 7,18(t,2H); 7,4-7,6(m,2H); 7,85(s,4H).

5-Aminooxy-1-(4-fluorphenyl)-1-pentin (Bsp. 2.6)

Zu einer Mischung aus 68 ml Ethanolamin und 40 ml Dichlormethan wurden portionsweise 47,7 g (0,148 mol) des oben dargestellten Phthalimidethers gegeben. Nach 2 h Rühren bei Raumtemperatur war eine klare Lösung entstanden. Diese wurde in 300 ml eiskalte, gesättigte Kochsalzlösung gegeben. Man extrahierte die Mischung dreimal mit 100 ml Dichlormethan, wusch die vereinigten organischen Phasen einmal mit Kochsalzlösung gegen, trocknete und engte ein. Die Titelverbindung fiel als Öl an. Ausbeute: 27,1 g (95%)
250-MHz-$^1$H-NMR($CDCl_3$):
$\delta$ (ppm) = 1,8-2,0(m,2H); 2,47(t,2H); 3,8(t,2H); 5,4(breites s, 2H); 6,9-7,1(m,2H); 7,3-7,45(m,2H).

1-Aminooxy-4-phenyl-2-butin (Bsp. 2.2)

1-Brom-4-phenyl-2-butin (Bsp. 1.8)

Zu einer Mischung aus 140,4 g (0,96 mol) 4-Phenyl-2-butin-1-ol (hergestellt nach G. Dupont, Bull. Soc, Chim. Fr., 1954, Seite 816) und 600 ml trockenem Toluol wurden 14,5 ml Pyridin zugegeben und anschließend 119,1 g (0,44 mol) Phosphortribromid innerhalb von 2 h zugetropft, wobei die Temperatur

nicht über 50 °C steigen durfte. Nach Rühren über Nacht bei Raumtemperatur goß man auf 1000 ml Eiswasser, trennte die organische Phase ab und extrahierte die wäßrige mehrfach mit Methyl-tert.-butylether. Die vereinigten organischen Phasen wurden anschließend neutral gewaschen und getrocknet. Das Rohprodukt, welches man nach Abziehen der Lösungsmittel im Vakuum erhielt, wurde fraktioniert destilliert. Ausbeute: 126 g mit einer Reinheit laut GC von 93%; Kp.: 83-85 °C bei 0,3 mbar.

N-(4-Phenyl-2-butinyloxy)phthalimid (Bsp. 1.9)

51,3 g (0,23 mol) des oben gewonnenen 1-Brom-4-phenyl-2-butins wurden innerhalb von 30 min zu einer Mischung, bestehend aus 230 ml N-Methylpyrrolidin-2-on, 3 g Kaliumjodid, 37 g (0,23 mol) N-Hydroxyphthalimid und 20,6 g (0,15 mol) Kaliumcarbonat, getropft. Man rührte noch 5 h bei 60 °C und dann über Nacht bei Raumtemperatur, goß in 800 ml Eiswasser, saugte die ausgefallenen Kristalle ab und wusch diese gründlich mit Wasser und Isopropanol. Ausbeute: 57 g (86%); Fp. von 127-129 °C.
250-MHz-$^1$H-NMR(DMSO-d6)
$\delta$ (ppm) = 3,71(t,2H); 4,95(t,2H); 7,28(s,5H); 7,9(s,4H)

1-Amino-oxy-4-phenyl-2-butin (Bsp. 2.2)

Zu einer Lösung von 28,7 g (0,099 mol) des oben hergestellten Phthalimidethers in 100 ml Essigester wurden 6 g (0,099 mol) Ethanolamin getropft. Man rührte 1,5 h bei 30 °C, kühlte im Eisbad, saugte die Kristalle ab und setzte der Mutterlauge eine Lösung von 8,9 g (0,099 mol) Oxalsäure in 130 ml Essigester zu. Dabei fiel die Titelverbindung als Oxalat aus. Man saugte die Kristalle ab, wusch diese mit kaltem Essigester und trocknete im Vakuum. Ausbeute: 20,2 g (81%), Fp. 113-117 °C
300 MHz-$^1$H-NMR (DMSO-d6)
$\delta$ (ppm) = 3,7(s,2H); 4,39(s,2H); 7,2-7,4(m,5H); 10,5(breites s)

Tabelle 1

| | X-A-Z | | | phys. Daten |
|---|---|---|---|---|
| | X-A | Z | X | $^1$H-NMR/ Fp [°C]/ Kp [°C/mbar] |
| 1.1 | X-$CH_2$-$CH_2$-C≡C- | 4-F-phenyl | OH | 2,65(t,2H);3,8 (t,2H);6,95(t,2H) |
| 1.2 | X-$CH_2$-$CH_2$-C≡C- | 4-Cl-phenyl | OH | 2,65(t,2H);3,8 (t,2H);7,35(d,2H) |
| 1.3 | X-$CH_2$-$CH_2$-C≡C- | Pyridin-3-yl | OH | 2,67(t,2H);3,8 (t,2H);8,45(d,1H) |
| 1.4 | X-$CH_2$-$CH_2$-$CH_2$-C≡C- | 4-F-phenyl | OH | 1,85(m,2H);2,5 (t,2H);6,95(t,2H) |
| 1.5 | X-$CH_2$-$CH_2$-$CH_2$-C≡C- | 4-Cl-phenyl | OH | 1,80(m,2H);2,5 (t,2H),7,35(d,2H) |
| 1.6 | X-$CH_2$-C≡C-$CH_2$- | 4-Cl-phenyl | Br | |
| 1.7 | X-$CH_2$-C≡C-$CH_2$- | 4-F-phenyl | Br | |
| 1.8 | X-$CH_2$-C≡C-$CH_2$- | 4-Phenyl | Br | 83-85/0,3 |
| 1.9 | X-$CH_2$-C≡C-$CH_2$- | 4-Phenyl | M | 127-129 |
| 1.10 | X-$CH_2$-$CH_2$-C≡C- | 4-F-phenyl | M | 87-88 |
| 1.11 | X-$CH_2$-$CH_2$-C≡C- | 4-Chlorphenyl | M | |
| 1.12 | X-$CH_2$-$CH_2$-C≡C- | Pyridin-3-yl | M | |
| 1.13 | X-$CH_2$-$CH_2$-$CH_2$-C≡C- | 4-F-phenyl | M | |
| 1.14 | X-$CH_2$-$CH_2$-$CH_2$-C≡C- | 4-Cl-phenyl | M | |
| 1.15 | X-$CH_2$-C≡C-$CH_2$- | 4-Cl-phenyl | M | |
| 1.16 | X-$CH_2$-C≡C-$CH_2$- | 4-F-phenyl | M | |
| 1.17 | X-$CH_2$-CH=C($CH_3$)-C≡C-*) | 4-Cl-phenyl | M | 109-111 |
| 1.18 | X-$CH_2$-CH=C($CH_3$)-C≡C-*) | 4-F-phenyl | M | |
| 1.19 | X-$CH_2$-CH=C($CH_3$)-C≡C-*) | 4-F-phenyl | OH | |
| 1.20 | X-$CH_2$-CH=C($CH_3$)-C≡C-*) | 4-Cl-phenyl | OH | |

M = 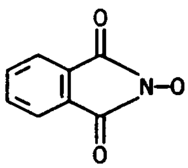

*) Z-Konfiguration an der Doppelbindung

Tabelle 2

| | $H_2N$-O-A-Z | | phys. Daten $^1$H-NMR/Fp [°C] |
|---|---|---|---|
| | O-A | Z | |
| 2.1 | $-O-CH_2-C{\equiv}C-$ | Phenyl | |
| 2.2 | $-O-CH_2-C{\equiv}C-CH_2-$ | Phenyl | 113-117 |
| 2.3 | $-O-CH_2-CH_2-C{\equiv}C-$ | 4-F-phenyl | 2,7(t);3,86(t); 5,5(s);6,9-7,05(m); 7,3-7,45(m) |
| 2.4 | $-O-CH_2-CH_2-C{\equiv}C-$ | 4-Cl-phenyl | |
| 2.5 | $-O-CH_2-CH_2-C{\equiv}C-$ | Pyridin-3-yl | |
| 2.6 | $-O-CH_2-CH_2-CH_2-C{\equiv}C-$ | 4-F-phenyl | 2,47(t);3,8 (t);5,4(s);6,9-7,1 (m);7,3-7,45(m) |
| 2.7 | $-O-CH_2-CH_2-CH_2-C{\equiv}C-$ | 4-Cl-phenyl | |
| 2.8 | $-O-CH_2-C{\equiv}C-CH_2-$ | 4-Cl-phenyl | |
| 2.9 | $-O-CH_2-C{\equiv}C-CH_2-$ | 4-Cl-phenyl | |
| 2.10 | $-O-CH_2-CH=C(CH_3)-C{\equiv}C-$*) | 4-F-phenyl | |
| 2.11 | $-O-CH_2-CH=C(CH_3)-C{\equiv}C-$*) | 4-Cl-phenyl | |
| 2.12 | $-O-CH_2-CH=C(CH_3)-C{\equiv}C-$**) | 4-F-phenyl | |
| 2.13 | $-O-CH_2-CH=C(CH_3)-C{\equiv}C-$**) | 4-Cl-phenyl | 54-56 |

*) E-Konfiguration an der Doppelbindung
**) Z-Konfiguration an der Doppelbindung

Die im nachstehenden Synthesebeispiel wiedergegebene Vorschrift wurde unter entsprechender Abwandlung der Ausgangsverbindung zur Gewinnung weiterer Verbindungen der Formel I benutzt; die erhaltenen Verbindungen sind in den nachfolgenden Tabellen mit physikalischen Angaben aufgeführt; Verbindungen ohne diese Angaben lassen sich aus den entsprechenden Stoffen in analoger Weise aufbauen. Sie lassen aufgrund ihrer nahen strukturellen Beziehungen zu den hergestellten und untersuchten Verbindungen eine gleichartige Wirkung erwarten.

Herstellvorschrift für 2-[1-(4-(4-Fluorphenyl)-but-3-inyloximino)-butyl-3-hydroxy-5-tetrahydropyran-4-yl-cyclohex-2-enon (Bsp. 3.20)

Zu einer Lösung von 4 g (15 mMol) 2-Butyryl-3-hydroxy-5-tetrahydropyran-4-yl-cyclohex-2-enon in 60 ml trockenem Methanol wurden 2,7 g (15 mMol) 4-(4-Fluorphenyl)-but-3-inoxymin gegeben. Nach 16 h Rühren bei Raumtemperatur wurde das Methanol im Wasserstrahlvakuum entfernt. Das Rohprodukt reinigte man mittels Chromatographie an Kieselgel (Laufmittel: Methylenchlorid). Ausbeute: 5,2 g (81,2%)

Tabelle 3

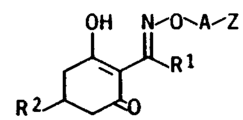

| Verb. Nr. | $R^2$ | $R^1$ | O-A | Z | $^1$H-NMR*) [δ ppm] | Fp. [°C] |
|---|---|---|---|---|---|---|
| 3.1 | Tetrahydrothiopyran-3-yl | Propyl | $-O-CH_2-C\equiv C-$ | Phenyl | 4,9(s,2H); 7,2-7,6(2m,5H) | |
| 3.2 | Tetrahydrothiopyran-3-yl | Propyl | $-O-CH_2-C\equiv C-CH_2-$ | Phenyl | 3,6(s,2H); 4,7(s,2H), 7,2-7,5(m,5H) | |
| 3.3 | Tetrahydrothiopyran-3-yl | Ethyl | $-O-CH_2-C\equiv C-CH_2-$ | Phenyl | 3,65 (s,2H); 4,7(s,2H); 7,2-7,5(m,5H) | |
| 3.4 | Tetrahydropyran-3-yl | Propyl | $-O-CH_2-C\equiv C-CH_2-$ | Phenyl | 3,65(s,2H); 4,7(s,2H); 7,2-7,5(m,5H) | |
| 3.5 | 2-Ethylthiopropyl | Propyl | $-O-CH_2-C\equiv C-$ | Phenyl | 4,9(s,2H); 7,3-7,6(m,5H) | |
| 3.6 | 2-Ethylthiopropyl | Propyl | $-O-CH_2-C\equiv C-CH_2-$ | Phenyl | 3,65(s,2H); 4,7(s,2H); 7,2-7,5(m,5H) | |
| 3.7 | Tetrahydropyran-4-yl | Ethyl | $-O-CH_2-C\equiv C-CH_2-$ | Phenyl | 3,65(s,2H); 4,7(s,2H); 7,2-7,5(m,5H) | |
| 3.8 | Tetrahydropyran-4-yl | Propyl | $-O-CH_2-C\equiv C-CH_2-$ | Phenyl | 3,6(s,2H); 4,65(s,2H); 7,1-7,6(m,5H) | |
| 3.9 | Tetrahydropyran-4-yl | Propyl | $-O-(CH_2)_3-C\equiv C-$ | 4-F-phenyl | 4,25(t,2H); 6,8-7,5(2m,4H) | |

*) ausgewählte Signale

Tabelle 3 (Fortsetzung)

| Verb. Nr. | $R^2$ | $R^1$ | O-A | Z | $^1$H-NMR*) [δ ppm] | Fp. [°C] |
|---|---|---|---|---|---|---|
| 3.10 | Tetrahydropyran-4-yl | Ethyl | $-O-(CH_2)_3-C{\equiv}C-$ | 4-F-phenyl | 4,25(t, 2H); 6,8-7,5(2m, 4H) | |
| 3.11 | Tetrahydrothiopyran-3-yl | Ethyl | $-O-(CH_2)_3-C{\equiv}C-$ | 4-F-phenyl | 4,25(t, 2H); 6,8-7,5(2m, 4H) | |
| 3.12 | Tetrahydrothiopyran-3-yl | Propyl | $-O-(CH_2)_3-C{\equiv}C-$ | 4-F-phenyl | 4,25(t, 2H); 6,8-7,5(2m, 4H) | |
| 3.13 | Tetrahydropyran-3-yl | Ethyl | $-O-(CH_2)_3-C{\equiv}C-$ | 4-F-phenyl | 4,25(t, 2H); 6,8-7,5(2m, 4H) | |
| 3.14 | Tetrahydropyran-3-yl | Propyl | $-O-(CH_2)_3-C{\equiv}C-$ | 4-F-phenyl | 4,25(t, 2H); 6,8-7,5(2m, 4H) | |
| 3.15 | Tetrahydrothiopyran-3-yl | Ethyl | $-O-CH_2-CH_2-C{\equiv}C-$ | 4-F-phenyl | 4.25(t); 6.98(dd); 7.35(dd) | 74- 90 |
| 3.16 | Tetrahydrothiopyran-3-yl | Propyl | $-O-CH_2-CH_2-C{\equiv}C-$ | 4-F-phenyl | 4.25(t); 6.98(dd); 7.35(dd) | - |
| 3.17 | Tetrahydropyran-3-yl | Ethyl | $-O-CH_2-CH_2-C{\equiv}C-$ | 4-F-phenyl | 4.25(t); 6.98(dd); 7.35(dd) | 55- 61 |
| 3.18 | Tetrahydropyran-3-yl | Propyl | $-O-CH_2-CH_2-C{\equiv}C-$ | 4-F-phenyl | 4.25(t); 6.98(dd); 7.35(dd) | - |
| 3.19 | Tetrahydropyran-4-yl | Ethyl | $-O-CH_2-CH_2-C{\equiv}C-$ | 4-F-phenyl | 4.25(t); 6.98(dd); 7.35(dd) | 83- 87 |
| 3.20 | Tetrahydropyran-4-yl | Propyl | $-O-CH_2-CH_2-C{\equiv}C-$ | 4-F-phenyl | 4.25(t); 6.98(dd); 7.35(dd) | 98-102 |

*) ausgewählte Signale

Tabelle 3 (Fortsetzung)

| Verb. Nr. | $R^2$ | $R^1$ | O-A | Z | $^1H$-NMR*) [δ ppm] | Fp. [°C] |
|---|---|---|---|---|---|---|
| 3.21 | 3-Isopropylisoxazol-5-yl | Propyl | $-O-CH_2-CH_2-C{\equiv}C-$ | 4-F-phenyl | 4.25(t); 5.94(s); 7.0(dd); 7.37(dd) | - |
| 3.22 | 4-Methylphenyl | Propyl | $-O-CH_2-CH_2-C{\equiv}C-$ | 4-F-phenyl | 4.25(t); 6.98(dd); 7.15(m); 7.35(dd) | 65-69 |
| 3.23 | 3,4-Dibromtetrahydropyran-3-yl | Propyl | $-O-CH_2-CH_2-C{\equiv}C-$ | 4-F-phenyl | 4.25(t); 6.98(dd); 7.35(dd) | - |
| 3.24 | Tetrahydrothiopyran-3-yl | Propyl | $-O-CH_2-CH_2-C{\equiv}C-$ | 4-Cl-phenyl | 4.25(t); 7.25(d); 7.35(d) | - |
| 3.25 | Tetrahydrothiopyran-3-yl | Ethyl | $-O-CH_2-CH_2-C{\equiv}C-$ | 4-Cl-phenyl | 4.25(t); 7.25(d); 7.35(d) | 82- 86 |
| 3.26 | Tetrahydropyran-3-yl | Ethyl | $-O-CH_2-CH_2-C{\equiv}C-$ | 4-Cl-phenyl | 4.25(t); 7.25(d); 7.35(d) | 99-101 |
| 3.27 | Tetrahydropyran-3-yl | Propyl | $-O-CH_2-CH_2-C{\equiv}C-$ | 4-Cl-phenyl | 4.25(t); 7.25(d); 7.35(d) | |
| 3.28 | Tetrahydropyran-4-yl | Ethyl | $-O-CH_2-CH_2-C{\equiv}C-$ | 4-Cl-phenyl | 4.25(t); 7.25(d); 7.35(d) | 98-101 |
| 3.29 | Tetrahydropyran-4-yl | Propyl | $-O-CH_2-CH_2-C{\equiv}C-$ | 4-Cl-phenyl | 4.25(t); 7.25(d); 7.35(d) | 115-118 |

*) ausgewählte Signale

Tabelle 3 (Fortsetzung)

| Verb. Nr. | $R^2$ | $R^1$ | O-A | Z | $^1$H-NMR*) [δ ppm] | Fp. [°C] |
|---|---|---|---|---|---|---|
| 3.30 | 3-Isopropylisoxazol-5-yl | Propyl | $-O-CH_2-CH_2-C{\equiv}C-$ | 4-Cl-phenyl | 4.25(t); 5.9(s); 7.25(d); 7.35(d) | 71- 74 |
| 3.31 | 4-Methylphenyl | Propyl | $-O-CH_2-CH_2-C{\equiv}C-$ | 4-Cl-phenyl | 4.25(t); 7.45(m); 7.28(M) | 93- 6 |
| 3.32 | 3,4-Dibromtetrahydropyran-3-yl | Propyl | $-O-CH_2-CH_2-C{\equiv}C-$ | 4-Cl-phenyl | 4.25(t); 7.25(d); 7.25(d) | - |
| 3.33 | Tetrahydrothiopyran-3-yl | Ethyl | $-O-(CH_2)_3-C{\equiv}C-$ | 4-Cl-phenyl | 1.15(t); 4.2(t); 7.25(d); 7.35(d) | |
| 3.34 | Tetrahydrothiopyran-3-yl | Propyl | $-O-(CH_2)_3-C{\equiv}C-$ | 4-Cl-phenyl | 0.98(t); 4.2(t); 7.25(d); 7.35(d) | |
| 3.35 | Tetrahydropyran-3-yl | Ethyl | $-O-(CH_2)_3-C{\equiv}C-$ | 4-Cl-phenyl | 1.15(t); 4.2(t); 7.25(d); 7.35(d) | |
| 3.36 | Tetrahydropyran-3-yl | Propyl | $-O-(CH_2)_3-C{\equiv}C-$ | 4-Cl-phenyl | 0.95(t); 4.2(t); 7.25(d); 7.35(d) | |

*) ausgewählte Signale

Tabelle 3 (Fortsetzung)

| Verb. Nr. | $R^2$ | $R^1$ | O-A | Z | $^1$H-NMR*) [δ ppm] | Fp. [°C] |
|---|---|---|---|---|---|---|
| 3.37 | Tetrahydropyran-4-yl | Ethyl | $-O-(CH_2)_3-C{\equiv}C-$ | 4-Cl-phenyl | 1.15(t); 4.2(t); 7.25(d); 7.35(d) | |
| 3.38 | Tetrahydropyran-4-yl | Propyl | $-O-(CH_2)_3-C{\equiv}C-$ | 4-Cl-phenyl | 0.98(t); 4.2(t); 7.25(d); 7.35(d) | |
| 3.39 | Tetrahydrothiopyran-3-yl | Ethyl | $-O-CH_2-CH_2-C{\equiv}C-$ | 2-Thienyl | 1.15(t); 2.75(t); 4.25(t); 7.05(m); 7.2(m) | |
| 3.40 | Tetrahydrothiopyran-3-yl | Propyl | $-O-CH_2-CH_2-C{\equiv}C-$ | 2-Thienyl | 0.95(t); 2.75(t); 4.25(t); 7.05(m); 7.2(m) | |
| 3.41 | Tetrahydropyran-3-yl | Ethyl | $-O-CH_2-CH_2-C{\equiv}C-$ | 2-Thienyl | 1.15(t); 2.75(t); 4.25(t); 7.05(m); 7.2(m) | |
| 3.42 | Tetrahydropyran-3-yl | Propyl | $-O-CH_2-CH_2-C{\equiv}C-$ | 2-Thienyl | 0.95(t); 2.75(t); 4.25(t); 7.05(m); 7.2(m) | |
| 3.43 | Tetrahydropyran-4-yl | Ethyl | $-O-CH_2-CH_2-C{\equiv}C-$ | 2-Thienyl | 1.15(t); 2.75(t); 4.25(t); 7.05(m); 7.2(m) | |
| 3.44 | Tetrahydropyran-4-yl | Propyl | $-O-CH_2-CH_2-C{\equiv}C-$ | 2-Thienyl | 0.95(t); 2.75(t); 4.25(t); 7.05(m); 7.2(m) | |

*) ausgewählte Signale

Tabelle 3 (Fortsetzung)

| Verb. Nr. | $R^2$ | $R^1$ | O-A | Z | $^1$H-NMR*) [δ ppm] | Fp. [°C] |
|---|---|---|---|---|---|---|
| 3.45 | Tetrahydrothiopyran-3-yl | Ethyl | $-O-CH_2-CH=C(CH_3)-C\equiv C-$ **) | 4-Cl-phenyl | 1.15(t); 2.0(s); 4.8(d); 5.9(t) | |
| 3.46 | Tetrahydrothiopyran-3-yl | Propyl | $-O-CH_2-CH=C(CH_3)-C\equiv C-$ **) | 4-Cl-phenyl | 0.95(t); 2.0(s); 4.8(d); 5.9(t) | |
| 3.47 | Tetrahydropyran-4-yl | Ethyl | $-O-CH_2-CH=C(CH_3)-C\equiv C-$ **) | 4-Cl-phenyl | 1.15(t); 2.0(s); 4.8(d); 5.9(t) | |
| 3.48 | Tetrahydropyran-4-yl | Propyl | $-O-CH_2-CH=C(CH_3)-C\equiv C-$ **) | 4-Cl-phenyl | 0.95(t); 2.0(s); 4.8(d); 5.9(t) | |
| 3.49 | 2-Ethylthiopropyl | Propyl | $-O-CH_2-CH=C(CH_3)-C\equiv C-$ **) | 4-Cl-phenyl | 0.95(t); 2.0(s); 4.8(d); 5.9(t) | |
| 3.50 | 2,4,6-Trimethylphenyl | Ethyl | $-O-CH_2-CH=C(CH_3)-C\equiv C-$ **) | 4-Cl-phenyl | 1.2(t); 2.0(s); 4.8(d); 5.95(t) | |
| 3.51 | Tetrahydrothiopropan--3-yl | Ethyl | $-O-CH_2-CH=C(CH_3)-C\equiv C-$ **) | 4-F-phenyl | 1.1(t); 2.0(s); 4.8(d); 5.9(t) | |
| 3.52 | Tetrahydrothiopropan--3-yl | Propyl | $-O-CH_2-CH=C(CH_3)-C\equiv C-$ **) | 4-F-phenyl | 0.95(t); 2.0(s); 4.8(d); 5.9(t) | |

*) ausgewählte Signale

**) Z-Konfiguration an der Doppelbindung

Tabelle 3 (Fortsetzung)

| Verb. Nr. | $R^2$ | $R^1$ | O-A | Z | $^1$H-NMR*) [δ ppm] | Fp. [°C] |
|---|---|---|---|---|---|---|
| 3.53 | Tetrahydropyran-4-yl | Propyl | $-O-CH_2-CH=C(CH_3)-C\equiv C-$ **) | 4-F-phenyl | 0.95(t); 2.0(s); 4.8(d); 5.9(t) | |
| 3.54 | Tetrahydropyran-4-yl | Ethyl | $-O-CH_2-CH=C(CH_3)-C\equiv C-$ **) | 4-F-phenyl | 1.15(t); 2.0(s); 4.8(d); 5.9(t) | |
| 3.55 | 2-Ethylthiopropyl | Propyl | $-O-CH_2-CH=C(CH_3)-C\equiv C-$ **) | 4-F-phenyl | 0.95(t); 2.0(s); 4.8(d); 5.9(t) | |

*) ausgewählte Signale
**) Z-Konfiguration an der Doppelbindung

## Anwendungsbeispiele

Die Wirkung der Cyclohexenonderivate der Formel I auf das Pflanzenwachstum läßt sich durch Gewächshausversuche zeigen:

Als Kulturgefäße dienten Plastikblumentöpfe mit lehmigem Sand mit etwa 3,0 % Humus als Substrat. Die Samen der Testpflanzen wurden nach Arten getrennt eingesät.

Bei Vorauflaufbehandlung wurden die in Wasser suspendierten oder emulgierten Wirkstoffe direkt nach Einsaat mittels fein verteilender Düsen aufgebracht. Die Gefäße wurden leicht beregnet, um Keimung und Wachstum zu fördern und anschließend mit durchsichtigen Plastikhauben abgedeckt, bis die Pflanzen angewachsen waren. Diese Abdeckung bewirkt ein gleichmäßiges Keimen der Testpflanzen, sofern dies nicht durch die Wirkstoffe beeinträchtigt wurde.

Zum Zwecke der Nachauflaufbehandlung wurden die Testpflanzen je nach Wuchsform erst bei einer Wuchshöhe von 3 bis 15 cm mit den in Wasser suspendierten oder emulgierten Wirkstoffen behandelt. Die Aufwandmenge für die Nachauflaufbehandlung betrug 0,25 kg/ha a.S.

Die Pflanzen wurden artenspezifisch bei Temperaturen von 10-25°C bzw. 20-35°C gehalten. Die Versuchsperiode erstreckte sich über 2 bis 4 Wochen. Während dieser Zeit wurden die Pflanzen gepflegt und ihre Reaktion auf die einzelnen Behandlungen wurde ausgewertet.

Bewertet wurde nach einer Skala von 0 bis 100. Dabei bedeutet 100 kein Aufgang der Pflanzen bzw. völlige Zerstörung zumindest der oberirdischen Teile und 0 keine Schädigung oder normaler Wachstumsverlauf.

Die in den Gewächshausversuchen verwendeten Pflanzen setzten sich aus folgenden Arten zusammen:

| Lateinischer Name | Deutscher Name | Englischer Name |
|---|---|---|
| Echinochloa crus-galli | Hühnerhirse | barnyard grass |
| Oryza sativa | Reis | rice |
| Setaria italica | Kolbenhirse | foxtail millet |
| Setaria viridis | Grüne Borstenhirse | green foxtail |

Mit 0,25 kg/ha a.S. im Nachauflaufverfahren eingesetzt, lassen sich mit den Verbindungen Nr. 3.2 und 3.4 unerwünschte grasartige Pflanzen sehr gut bekämpfen, bei gleichzeitiger guter Verträglichkeit an der Beispielkultur Reis.

## Patentansprüche

## Patentansprüche für folgende Vertragsstaaten : AT, BE, CH, DE, FR, GB, IT, LI, NL

**1.** Cyclohexenonoximether der allgemeinen Formel I

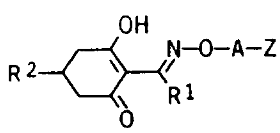

(I)

in der die Variablen folgende Bedeutung haben:

- $R^1$ eine $C_1$-$C_6$-Alkylgruppe;
- A eine unsubstituierte oder durch 1 bis 3 $C_1$-$C_3$-Alkylgruppen, Halogenatome substituierte $C_3$-$C_6$-Alkinylenkette;
- Z Phenyl, 5-gliedriger oder 6-gliedriger Heteroaromat mit einem bis drei Heteroatomen, ausgewählt aus einer Gruppe bestehend aus drei Stickstoffatomen und einem Sauerstoff- oder Schwefelatom, wobei die aromatischen Reste unsubstituiert oder mit n gleichen oder verschiedenen Resten X substituiert sein können;
- X Nitro, Cyano, Halogen, $C_1$-$C_4$-Alkyl, $C_1$-$C_4$-Alkoxy, $C_1$-$C_4$-Alkylthio, $C_1$-$C_4$-Halogenalkyl, $C_1$-$C_4$-Halogenalkoxy, Carboxyl, $C_1$-$C_4$-Alkoxycarbonyl, Benzyloxycarbonyl, Phenyl, wobei die aromatischen Reste noch einen bis drei Substituenten tragen können, ausgewählt aus einer Gruppe bestehend aus Nitro, Cyano, Halogen, $C_1$-$C_4$-Alkyl, $C_1$-$C_4$-Alkoxy, $C_1$-$C_4$-Alkylthio, $C_1$-$C_4$-Halogenalkyl, $C_1$-$C_4$-Halogenalkoxy, Carboxyl, $C_1$-$C_4$-Alkoxycarbonyl- und Benzyloxycarbonyl;
- n 0 bis 3 oder 1 bis 5 für den Fall, daß X Halogenatome bedeutet;
- $R^2$ eine $C_1$-$C_4$-Alkoxy-$C_1$-$C_6$-alkyl- oder $C_1$-$C_4$-Alkylthio-$C_1$-$C_6$-alkylgruppe;
  eine $C_3$-$C_7$-Cycloalkylgruppe oder eine $C_5$-$C_7$-Cycloalkenylgruppe, wobei diese Gruppen noch einen bis drei Reste tragen können, ausgewählt aus einer Gruppe bestehend aus $C_1$-

$C_4$-Alkyl, $C_1$-$C_4$-Alkoxy, $C_1$-$C_4$-Alkylthio, $C_1$-$C_4$-Halogenalkyl, Hydroxy und Halogen;
ein 5-gliedriger gesättigter Heterocyclus der ein oder zwei Heteroatome enthält, ausgewählt aus einer Gruppe bestehend aus Sauerstoff und Schwefel, und der noch einen bis drei Reste tragen kann, ausgewählt aus einer Gruppe bestehend aus $C_1$-$C_4$-Alkyl, $C_1$-$C_4$-Alkoxy, $C_1$-$C_4$-Alkylthio und $C_1$-$C_4$-Halogenalkyl,
ein 6- oder 7-gliedriger gesättigter oder ein- oder zweifach ungesättigter Heterocyclus, enthaltend ein oder zwei Heteroatome, ausgewählt aus einer Gruppe bestehend aus Sauerstoff und Schwefel, wobei der Heterocyclus noch einen bis drei Reste tragen kann, ausgewählt aus einer Gruppe bestehend aus Hydroxy, Halogen, $C_1$-$C_4$-Alkyl, $C_1$-$C_4$-Alkoxy, $C_1$-$C_4$-Alkylthio und $C_1$-$C_4$-Halogenalkyl;
ein 5-gliedriger Heteroaromat, enthaltend ein bis drei Heteroatome, ausgewählt aus einer Gruppe bestehend aus zwei Stickstoffatomen und einen Sauerstoff- oder Schwefelatom, wobei der Heterocyclus noch einen bis drei Reste tragen kann, ausgewählt aus einer Gruppe bestehend aus Halogen, Cyano, $C_1$-$C_4$-Alkyl, $C_1$-$C_4$-Alkoxy, $C_1$-$C_4$-Alkylthio, $C_1$-$C_4$-Halogenalkyl, $C_2$-$C_6$-Alkenyl, $C_2$-$C_6$-Alkenyloxy, $C_2$-$C_6$-Halogenalkenyl und $C_1$-$C_4$-Alkoxy-$C_1$-$C_4$-alkyl,
die Phenylgruppe oder die Pyridylgruppe, wobei diese Gruppen noch einen bis drei Reste tragen können, ausgewählt aus einer Gruppe bestehend aus Halogen, Nitro, Cyano, $C_1$-$C_4$-Alkyl, $C_1$-$C_4$-Alkoxy, $C_1$-$C_4$-Alkylthio, $C_1$-$C_4$-Halogenalkyl, $C_3$-$C_6$-Alkenyloxy, $C_3$-$C_6$-Alkinyloxy und $-NR^3R^4$, worin

$R^3$ Wasserstoff, eine $C_1$-$C_4$-Alkylgruppe, eine $C_3$-$C_6$-Alkenylgruppe oder eine $C_3$-$C_6$-Alkinylgruppe und

$R^4$ Wasserstoff, eine $C_1$-$C_4$-Alkylgruppe, eine $C_3$-$C_6$-Alkenylgruppe, eine $C_3$-$C_6$-Alkinylgruppe, eine $C_1$-$C_6$-Acylgruppe oder ein Benzoylrest, wobei der aromatische Ring zusätzlich einen bis drei Substituenten tragen kann, ausgewählt aus einer Gruppe bestehend aus Nitro, Cyano, Halogen, $C_1$-$C_4$-Alkyl, $C_1$-$C_4$-Alkoxy, $C_1$-$C_4$-Alkylthio und $C_1$-$C_4$-Halogenalkyl
bedeuten,

sowie ihre landwirtschaftlich nutzbaren Salze und Ester von $C_1$-$C_{10}$-Carbonsäuren und anorganischen Säuren.

**2.** Verfahren zur Herstellung von Verbindungen der Formel I gemäß Anspruch 1, dadurch gekennzeichnet, daß man ein Cyclohexenon der Formel II

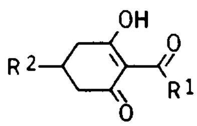

II

in an sich bekannter Weise in einem inerten organischen Lösungsmittel mit einem Hydroxylamin der Formel III

$H_2N$-O-A-Z III,

oder einem Salz des entsprechenden Hydroxylamins, umsetzt, und das Verfahrensprodukt I gewünschtenfalls in seine landwirtschaftlich nutzbares Salz oder in einem Ester von $C_1$-$C_{10}$-Carbonsäuren oder eine anorganische Säure überführt.

**3.** Herbizides Mittel, enthaltend inerte Zusatzstoffe und eine herbizid wirksame Menge mindestens einer Verbindung der Formel I gemäß Anspruch 1.

**4.** Verfahren zur Bekämpfung unerwünschten Pflanzenwuchses, dadurch gekennzeichnet, daß man die unerwünschten Pflanzen und/oder ihren Lebensraum mit einer herbizid wirksamen Menge eines Cyclohexenonderivates der Formel I gemäß Anspruch 1 behandelt.

**5.** Hydroxylamine der Formel III

$H_2N$-O-A-Z III,

in der A und Z die folgende Bedeutung haben:

A eine unsubstituierte oder durch 1 bis 3 $C_1$-$C_3$-Alkylgruppen, Halogenatomen substituierte $C_3$-$C_6$-Alkinylenkette;

Z Phenyl, 5-gliedriger oder 6-gliedriger Heteroaromat mit einem oder mehreren Heteroatomen aus der Gruppe Sauerstoff, Schwefel oder Stickstoff, wobei die aromatischen Reste unsubstituiert oder mit n gleichen oder verschiedenen Resten X substituiert sein können;

X bedeutet Nitro, Cyano, Halogen, $C_1$-$C_4$-Alkyl, $C_1$-$C_4$-Alkoxy, $C_1$-$C_4$-Alkylthio, $C_1$-$C_4$-Halogenalkyl, $C_1$-$C_4$-Halogenalkoxy, Carboxyl, $C_1$-$C_4$-Alkoxycarbonyl, Benzyloxycarbonyl und/oder Phenyl, wobei die aromatischen Reste ein bis drei der folgenden Substituenten tragen können: Nitro, Cyano, Halogen, $C_1$-$C_4$-Alkyl, $C_1$-$C_4$-Alkoxy, $C_1$-$C_4$-Alkylthio, $C_1$-$C_4$-Halogenalkyl, $C_1$-$C_4$-Halogenalkoxy, Carboxyl, $C_1$-$C_4$-Alkoxycarbonyl und/oder Benzyloxycarbonyl;

n bedeutet 0 bis 3 oder 1 bis 5 für den Fall, daß X Halogen ist.

**6.** Verfahren zur Herstellung von Hydroxylaminen der Formel III, gemäß Anspruch 5, dadurch gekennzeichnet, daß man eine Verbindung der Formel IV

X-A-Z IV,

in der A und Z die in Anspruch 5 genannte Bedeutung haben und X für eine nukleophil verdrängbare Abgangsgruppe steht, mit einem cyclischen Hydroxyimid der Formel V

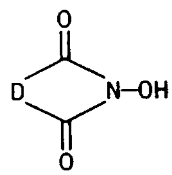

V,

in der D für $C_2$- und $C_3$-Alkylen, $C_2$-Alkenylen oder einen Fünf- oder Sechsring steht, der 1 bis 3 Doppelbindungen und ein Stickstoffatom enthalten kann, in Gegenwart einer Base zu Verbindungen der Formel VI

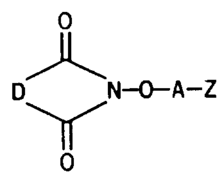

VI,

umsetzt und aus den Imidethern VI die Hydroxylamine III mittels Basen freisetzt.

**7.** Verfahren nach Anspruch 6, dadurch gekennzeichnet, daß man das Hydroxylamin III aus dem Imidether VI mittels Ethanolamin freisetzt.

**Patentansprüche für folgenden Vertragsstaat : ES**

**1.** Verfahren zur Herstellung von Cyclohexenonoximether der allgemeinen Formel I

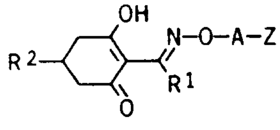

(I)

in der die Variablen folgende Bedeutung haben:

$R^1$ eine $C_1$-$C_6$-Alkylgruppe;

A eine unsubstituierte oder durch 1 bis 3 $C_1$-$C_3$-Alkylgruppen, Halogenatome substituierte $C_3$-$C_6$-Alkinylenkette;

Z Phenyl, 5-gliedriger oder 6-gliedriger Heteroaromat mit einem bis drei Heteroatomen, ausgewählt aus einer Gruppe bestehend aus drei Stickstoffatomen und einem Sauerstoff- oder Schwefelatom, wobei die aromatischen Reste unsubstituiert oder mit n gleichen oder verschiedenen Resten X substituiert sein können;

X Nitro, Cyano, Halogen, $C_1$-$C_4$-Alkyl, $C_1$-$C_4$-Alkoxy, $C_1$-$C_4$-Alkylthio, $C_1$-$C_4$-Halogenalkyl, $C_1$-$C_4$-Halogenalkoxy, Carboxyl, $C_1$-$C_4$-Alkoxycarbonyl, Benzyloxycarbonyl, Phenyl, wobei die aromatischen Reste noch einen bis drei Substituenten tragen können, ausgewählt aus einer Gruppe bestehend aus Nitro, Cyano, Halogen, $C_1$-$C_4$-Alkyl, $C_1$-$C_4$-Alkoxy, $C_1$-$C_4$-Alkylthio, $C_1$-$C_4$-Halogenalkyl, $C_1$-$C_4$-Halogenalkoxy, Carboxyl, $C_1$-$C_4$-Alkoxycarbonyl- und Benzyloxycarbonyl;

n 0 bis 3 oder 1 bis 5 für den Fall, daß X Halogenatome bedeutet;

$R^2$ eine $C_1$-$C_4$-Alkoxy-$C_1$-$C_6$-alkyl- oder $C_1$-$C_4$-Alkylthio-$C_1$-$C_6$-alkylgruppe;
eine $C_3$-$C_7$-Cycloalkylgruppe oder eine $C_5$-$C_7$-Cycloalkenylgruppe, wobei diese Gruppen noch einen bis drei Reste tragen können, ausgewählt aus einer Gruppe bestehend aus $C_1$-$C_4$-Alkyl, $C_1$-$C_4$-Alkoxy, $C_1$-$C_4$-Alkylthio, $C_1$-$C_4$-Halogenalkyl, Hydroxy und Halogen;
ein 5-gliedriger gesättigter Heterocyclus der ein oder zwei Heteroatome enthält, ausgewählt aus einer Gruppe bestehend aus Sauerstoff und Schwefel, und der noch einen bis drei Reste tragen kann, ausgewählt aus einer Gruppe bestehend aus $C_1$-$C_4$-Alkyl, $C_1$-$C_4$-Alkoxy, $C_1$-$C_4$-Alkylthio und $C_1$-$C_4$-Halogenalkyl,
ein 6- oder 7-gliedriger gesättigter oder ein- oder zweifach ungesättigter Heterocyclus, enthaltend ein oder zwei Heteroatome, ausgewählt aus einer Gruppe bestehend aus Sauerstoff und Schwefel, wobei der Heterocyclus noch einen bis drei Reste tragen kann, ausgewählt aus einer Gruppe bestehend aus Hydroxy, Halogen, $C_1$-$C_4$-Alkyl, $C_1$-$C_4$-Alkoxy, $C_1$-$C_4$-Alkylthio und $C_1$-$C_4$-Halogenalkyl;
ein 5-gliedriger Heteroaromat, enthaltend ein bis drei Heteroatome, ausgewählt aus einer Gruppe bestehend aus zwei Stickstoffatomen und einen Sauerstoff- oder Schwefelatom, wobei der Heterocyclus noch einen bis drei Reste tragen kann, ausgewählt aus einer Gruppe bestehend aus Halogen, Cyano, $C_1$-$C_4$-Alkyl, $C_1$-$C_4$-Alkoxy, $C_1$-$C_4$-Alkylthio, $C_1$-$C_4$-Halogenalkyl, $C_2$-$C_6$-Alkenyl, $C_2$-$C_6$-Alkenyloxy, $C_2$-$C_6$-Halogenalkenyl und $C_1$-$C_4$-Alkoxy-$C_1$-$C_4$-alkyl,
die Phenylgruppe oder die Pyridylgruppe, wobei diese Gruppen noch einen bis drei Reste tragen können, ausgewählt aus einer Gruppe bestehend aus Halogen, Nitro, Cyano, $C_1$-$C_4$-Alkyl, $C_1$-$C_4$-Alkoxy, $C_1$-$C_4$-Alkylthio, $C_1$-$C_4$-Halogenalkyl, $C_3$-$C_6$-Alkenyloxy, $C_3$-$C_6$-Alkinyloxy und $-NR^3R^4$, worin

$R^3$ Wasserstoff, eine $C_1$-$C_4$-Alkylgruppe, eine $C_3$-$C_6$-Alkenylgruppe oder eine $C_3$-$C_6$-Alkinylgruppe und

$R^4$ Wasserstoff, eine $C_1$-$C_4$-Alkylgruppe, eine $C_3$-$C_6$-Alkenylgruppe, eine $C_3$-$C_6$-Alkinylgruppe, eine $C_1$-$C_6$-Acylgruppe oder ein Benzoylrest, wobei der aromatische Ring zusätzlich einen bis drei Substituenten tragen kann, ausgewählt aus einer Gruppe bestehend aus Nitro, Cyano, Halogen, $C_1$-$C_4$-Alkyl, $C_1$-$C_4$-Alkoxy, $C_1$-$C_4$-Alkylthio und $C_1$-$C_4$-Halogenalkyl
bedeuten,

sowie deren landwirtschaftlich nutzbaren Salzen und Estern von $C_1$-$C_{10}$-Carbonsäuren und anorganischen Säuren, dadurch gekennzeichnet, daß man ein Cyclohexenon der Formel II

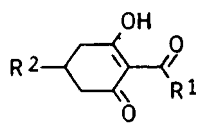

II

in an sich bekannter Weise in einem inerten organischen Lösungsmittel mit einem Hydroxylamin der Formel III

$H_2N$-O-A-Z III,

oder einem Salz des entsprechenden Hydroxylamins, umsetzt, und das Verfahrensprodukt I gewünschtenfalls in seine landwirtschaftlich nutzbares Salz oder in einem Ester von $C_1$-$C_{10}$-Carbonsäuren oder

eine anorganische Säure überführt.

2. Herbizides Mittel, enthaltend inerte Zusatzstoffe und eine herbizid wirksame Menge mindestens einer Verbindung der Formel I gemäß Anspruch 1.

3. Verfahren zur Bekämpfung unerwünschten Pflanzenwuchses, dadurch gekennzeichnet, daß man die unerwünschten Pflanzen und/oder ihren Lebensraum mit einer herbizid wirksamen Menge eines Cyclohexenon-derivates der Formel I gemäß Anspruch 1 behandelt.

4. Verfahren zur Herstellung von Hydroxylaminen der Formel III,

$H_2N$-O-A-Z III,

in der A und Z die folgende Bedeutung haben:

A eine unsubstituierte oder durch 1 bis 3 $C_1$-$C_3$-Alkylgruppen, Halogenatomen substituierte $C_3$-$C_6$-Alkinylenkette;

Z Phenyl, 5-gliedriger oder 6-gliedriger Heteroaromat mit einem oder mehreren Heteroatomen aus der Gruppe Sauerstoff, Schwefel oder Stickstoff, wobei die aromatischen Reste unsubstituiert oder mit n gleichen oder verschiedenen Resten X substituiert sein können;

X bedeutet Nitro, Cyano, Halogen, $C_1$-$C_4$-Alkyl, $C_1$-$C_4$-Alkoxy, $C_1$-$C_4$-Alkylthio, $C_1$-$C_4$-Halogenalkyl, $C_1$-$C_4$-Halogenalkoxy, Carboxyl, $C_1$-$C_4$-Alkoxycarbonyl, Benzyloxycarbonyl und/oder Phenyl, wobei die aromatischen Reste ein bis drei der folgenden Substituenten tragen können: Nitro, Cyano, Halogen, $C_1$-$C_4$-Alkyl, $C_1$-$C_4$-Alkoxy, $C_1$-$C_4$-Alkylthio, $C_1$-$C_4$-Halogenalkyl, $C_1$-$C_4$-Halogenalkoxy, Carboxyl, $C_1$-$C_4$-Alkoxycarbonyl und/oder Benzyloxycarbonyl;

n bedeutet 0 bis 3 oder 1 bis 5 für den Fall, daß X Halogen ist,

dadurch gekennzeichnet, daß man eine Verbindung der Formel IV

X-A-Z IV,

in der A und Z die obengenannte Bedeutung haben und X für eine nukleophil verdrängbare Abgangsgruppe steht, mit einem cyclischen Hydroxyimid der Formel V

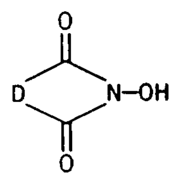

V,

in der D für $C_2$- und $C_3$-Alkylen, $C_2$-Alkenylen oder einen Fünf- oder Sechsring steht, der 1 bis 3 Doppelbindungen und ein Stickstoffatom enthalten kann, in Gegenwart einer Base zu Verbindungen der Formel VI

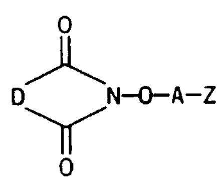

VI,

umsetzt und aus den Imidethern VI die Hydroxylamine III mittels Basen freisetzt.

5. Verfahren nach Anspruch 4, dadurch gekennzeichnet, daß man das Hydroxylamin III aus dem Imidether VI mittels Ethanolamin freisetzt.

## Claims

**Claims for the following Contracting States : AT, BE, CH, DE, FR, GB, IT, LI, NL**

**1.** A cyclohexenone oxime ether of the formula I

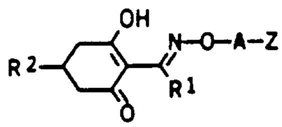

(I)

where

| | |
|---|---|
| $R^1$ | is $C_1$-$C_6$-alkyl; |
| A | is a $C_3$-$C_6$-alkynylene chain which is unsubstituted or substituted by 1 to 3 $C_1$-$C_3$-alkyl groups or halogen atoms; |
| Z | is phenyl or a 5-membered or 6-membered heteroaromatic structure having one to three hetero atoms selected from the group consisting of three nitrogen atoms and one oxygen or sulfur atom, where the aromatic radicals may be unsubstituted or substituted by n identical or different radicals X; |
| X | is nitro, cyano, halogen, $C_1$-$C_4$-alkyl, $C_1$-$C_4$-alkoxy, $C_1$-$C_4$-alkylthio, $C_1$-$C_4$-haloalkyl, $C_1$-$C_4$-haloalkoxy, carboxyl, $C_1$-$C_4$-alkoxycarbonyl, benzyloxycarbonyl or phenyl, where the aromatic radicals may furthermore carry one to three substituents selected from the group consisting of nitro, cyano, halogen, $C_1$-$C_4$-alkyl,$C_1$-$C_4$-alkoxy, $C_1$-$C_4$-alkylthio, $C_1$-$C_4$-haloalkyl, $C_1$-$C_4$-haloalkoxy, carboxyl, $C_1$-$C_4$-alkoxycarbonyl and benzyloxycarbonyl; |
| n | is from 0 to 3, or from 1 to 5 where X is halogen, and |
| $R^2$ | is $C_1$-$C_4$-alkoxy-$C_1$-$C_6$-alkyl or $C_1$-$C_4$-alkylthio-$C_1$-$C_6$-alkyl;<br>$C_3$-$C_7$-cycloalkyl or $C_5$-$C_7$-cycloalkenyl, where these groups may furthermore carry one to three radicals selected from the group consisting of $C_1$-$C_4$-alkyl, $C_1$-$C_4$-alkoxy, $C_1$-$C_4$-alkylthio, $C_1$-$C_4$-haloalkyl, hydroxyl and halogen;<br>a 5-membered saturated heterocyclic structure which contains one or two hetero atoms selected from the group consisting of oxygen and sulfur and which may furthermore carry one to three radicals selected from the group consisting of $C_1$-$C_4$-alkyl, $C_1$-$C_4$-alkoxy, $C_1$-$C_4$-alkylthio and $C_1$-$C_4$-haloalkyl;<br>a 6-membered or 7-membered saturated or monounsaturated or diunsaturated heterocyclic structure containing one or two hetero atoms selected from the group consisting of oxygen and sulfur, where the heterocyclic structure may furthermore carry one to three radicals selected from the group consisting of hydroxyl, halogen, $C_1$-$C_4$-alkyl, $C_1$-$C_4$-alkoxy, $C_1$-$C_4$-alkylthio and $C_1$-$C_4$-haloalkyl;<br>a 5-membered heteroaromatic structure containing one to three hetero atoms selected from the group consisting of two nitrogen atoms and one oxygen or sulfur atom, where the heterocyclic structure may furthermore carry one to three radicals selected from the group consisting of halogen, cyano, $C_1$-$C_4$-alkyl, $C_1$-$C_4$-alkoxy, $C_1$-$C_4$-alkylthio, $C_1$-$C_4$-haloalkyl, $C_2$-$C_6$-alkenyl, $C_2$-$C_6$-alkenyloxy, $C_2$-$C_6$-haloalkenyl and $C_1$-$C_4$-alkoxy-$C_1$-$C_4$-alkyl, or<br>phenyl or pyridyl, where these groups may furthermore carry one to three radicals selected from the group consisting of halogen, nitro, cyano, $C_1$-$C_4$-alkyl,$C_1$-$C_4$-alkoxy, $C_1$-$C_4$-alkylthio, $C_1$-$C_4$-haloalkyl, $C_3$-$C_6$-alkenyloxy, $C_3$-$C_6$-alkynyloxy and $-NR^3R^4$, where |
| $R^3$ | is hydrogen, $C_1$-$C_4$-alkyl, $C_3$-$C_6$-alkenyl or $C_3$-$C_6$-alkynyl and |
| $R^4$ | is hydrogen, $C_1$-$C_4$-alkyl, $C_3$-$C_6$-alkenyl, $C_3$-$C_6$-alkynyl, $C_1$-$C_6$-acyl or benzoyl, where the aromatic ring may additionally carry one to three substituents selected from the group consisting of nitro, cyano, halogen, $C_1$-$C_4$-alkyl, $C_1$-$C_4$-alkoxy, $C_1$-$C_4$-alkylthio and $C_1$-$C_4$-haloalkyl, |

and its agriculturally useful salts and esters of $C_1$-$C_{10}$-carboxylic acids and inorganic acids.

**2.** A process for the preparation of a compound of the formula I as claimed in claim 1, wherein a cyclohexenone of the formula II

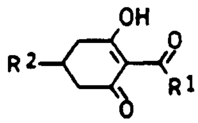

II

is reacted with a hydroxylamine of the formula III

$H_2N$-O-A-Z III

or with a salt of the corresponding hydroxylamine in a conventional manner in an inert organic solvent, and the product I is, if desired, converted into its agriculturally useful salt or into an ester of a $C_1$-$C_{10}$-carboxylic acid or an inorganic acid.

**3.** A herbicide containing inert additives and a herbicidal amount of one or more compounds of the formula I as claimed in claim 1.

**4.** A method for controlling undesirable plant growth, wherein the undesirable plants or their habitat is or are treated with a herbicidal amount of a cyclohexenone derivative of the formula I as claimed in claim 1.

**5.** A hydroxylamine of the formula III

$H_2N$-O-A-Z III

where

A is a $C_3$-$C_6$-alkynylene chain which is unsubstituted or substituted by 1 to 3 $C_1$-$C_3$-alkyl groups or halogen atoms;

Z is phenyl, or a 5-membered or 6-membered heteroaromatic structure having one or more hetero atoms selected from the group consisting of oxygen, sulfur and nitrogen, where the aromatic radicals may be unsubstituted or substituted by n identical or different radicals X;

X is nitro, cyano, halogen, $C_1$-$C_4$-alkyl, $C_1$-$C_4$-alkoxy, $C_1$-$C_4$-alkylthio, $C_1$-$C_4$-haloalkyl, $C_1$-$C_4$-haloalkoxy, $C_1$-$C_4$-alkylthio, $C_1$-$C_4$-haloalkyl, $C_1$-$C_4$-haloalkoxy, carboxyl, $C_1$-$C_4$-alkoxycarbonyl, benzyloxycarbonyl or phenyl, where the aromatic radicals may carry one to three of the following substituents: nitro, cyano, halogen, $C_1$-$C_4$-alkyl, $C_1$-$C_4$-alkoxy, $C_1$-$C_4$-alkylthio, $C_1$-$C_4$-haloalkyl, $C_1$-$C_4$-haloalkoxy, carboxyl, $C_1$-$C_4$-alkoxycarbonyl, or benzyloxycarbonyl and

n is from 0 to 3, or from 1 to 5 where X is halogen.

**6.** A process for the preparation of a hydroxylamine of the formula III, as claimed in claim 5, wherein a compound of the formula IV

X-A-Z IV

where A and Z have the meanings stated in claim 5 and X is a nucleophilically substitutable leaving group, is reacted with a cyclic hydroximide of the formula V

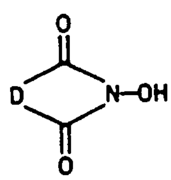

V,

where D is $C_2$- or $C_3$-alkylene, $C_2$-alkenylene or a five-membered or six-membered ring which may contain 1 to 3 double bonds and one nitrogen atom, in the presence of a base to give a compound of the formula VI

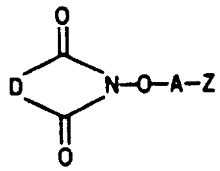

VI,

and the hydroxylamine III is liberated from the imide ether VI by means of a base.

**7.** A process as claimed in claim 6, wherein the hydroxylamine III is liberated from the imide ether VI by means of ethanolamine.

**Claims for the following Contracting State : ES**

**1.** A process for the preparation of a cyclohexenone oxime ether of the formula I

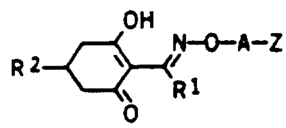

(I)

where

$R^1$ is $C_1$-$C_6$-alkyl;

A is a $C_3$-$C_6$-alkynylene chain which is unsubstituted or substituted by 1 to 3 $C_1$-$C_3$-alkyl groups or halogen atoms;

Z is phenyl or a 5-membered or 6-membered heteroaromatic structure having one to three hetero atoms selected from the group consisting of three nitrogen atoms and one oxygen or sulfur atom, where the aromatic radicals may be unsubstituted or substituted by n identical or different radicals X;

X is nitro, cyano, halogen, $C_1$-$C_4$-alkyl, $C_1$-$C_4$-alkoxy, $C_1$-$C_4$-alkylthio, $C_1$-$C_4$-haloalkyl, $C_1$-$C_4$-haloalkoxy, carboxyl, $C_1$-$C_4$-alkoxycarbonyl, benzyloxycarbonyl or phenyl, where the aromatic radicals may furthermore carry one to three substituents selected from the group consisting of nitro, cyano, halogen, $C_1$-$C_4$-alkyl,$C_1$-$C_4$-alkoxy, $C_1$-$C_4$-alkylthio, $C_1$-$C_4$-haloalkyl, $C_1$-$C_4$-haloalkoxy, carboxyl, $C_1$-$C_4$-alkoxycarbonyl and benzyloxycarbonyl;

n is from 0 to 3, or from 1 to 5 where X is halogen, and

$R^2$ is $C_1$-$C_4$-alkoxy-$C_1$-$C_6$-alkyl or $C_1$-$C_4$-alkylthio-$C_1$-$C_6$-alkyl;

$C_3$-$C_7$-cycloalkyl or $C_5$-$C_7$-cycloalkenyl, where these groups may furthermore carry one to three radicals selected from the group consisting of $C_1$-$C_4$-alkyl, $C_1$-$C_4$-alkoxy, $C_1$-$C_4$-alkylthio, $C_1$-$C_4$-haloalkyl, hydroxyl and halogen;

a 5-membered saturated heterocyclic structure which contains one or two hetero atoms selected from the group consisting of oxygen and sulfur and which may furthermore carry one to three radicals selected from the group consisting of $C_1$-$C_4$-alkyl, $C_1$-$C_4$-alkoxy, $C_1$-$C_4$-alkylthio and $C_1$-$C_4$-haloalkyl;

a 6-membered or 7-membered saturated or monounsaturated or diunsaturated heterocyclic structure containing one or two hetero atoms selected from the group consisting of oxygen and sulfur, where the heterocyclic structure may furthermore carry one to three radicals selected from the group consisting of hydroxyl, halogen, $C_1$-$C_4$-alkyl, $C_1$-$C_4$-alkoxy, $C_1$-$C_4$-alkylthio and $C_1$-$C_4$-haloalkyl;

a 5-membered heteroaromatic structure containing one to three hetero atoms selected from the group consisting of two nitrogen atoms and one oxygen or sulfur atom, where the heterocyclic structure may furthermore carry one to three radicals selected from the group consisting of halogen, cyano, $C_1$-$C_4$-alkyl, $C_1$-$C_4$-alkoxy, $C_1$-$C_4$-alkylthio, $C_1$-$C_4$-haloalkyl, $C_2$-$C_6$-alkenyl, $C_2$-$C_6$-alkenyloxy, $C_2$-$C_6$-haloalkenyl and $C_1$-$C_4$-alkoxy-$C_1$-$C_4$-alkyl, or

phenyl or pyridyl, where these groups may furthermore carry one to three radicals selected from the group consisting of halogen, nitro, cyano, $C_1$-$C_4$-alkyl,$C_1$-$C_4$-alkoxy, $C_1$-$C_4$-alkylthio, $C_1$-$C_4$-haloalkyl, $C_3$-$C_6$-alkenyloxy, $C_3$-$C_6$-alkynyloxy and $-NR^3R^4$, where

$R^3$ is hydrogen, $C_1$-$C_4$-alkyl, $C_3$-$C_6$-alkenyl or $C_3$-$C_6$-alkynyl and

$R^4$ is hydrogen, $C_1$-$C_4$-alkyl, $C_3$-$C_6$-alkenyl, $C_3$-$C_6$-alkynyl, $C_1$-$C_6$-acyl or benzoyl, where the aromatic ring may additionally carry one to three substituents selected from the group consisting of nitro, cyano, halogen, $C_1$-$C_4$-alkyl, $C_1$-$C_4$-alkoxy, $C_1$-$C_4$-alkylthio and $C_1$-$C_4$-haloalkyl,

and its agriculturally useful salts and esters of $C_1$-$C_{10}$-carboxylic acids and inorganic acids, wherein a cyclohexenone of the formula II

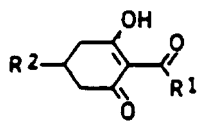

II

is reacted with a hydroxylamine of the formula III

$H_2N$-O-A-Z    III

or with a salt of the corresponding hydroxylamine in a conventional manner in an inert organic solvent, and the product I is, if desired, converted into its agriculturally useful salt or into an ester of a $C_1$-$C_{10}$-carboxylic acid or an inorganic acid.

**2.** A herbicide containing inert additives and a herbicidal amount of one or more compounds of the formula I as claimed in claim 1.

**3.** A method for controlling undesirable plant growth, wherein the undesirable plants or their habitat is or are treated with a herbicidal amount of a cyclohexenone derivative of the formula I as claimed in claim 1.

**4.** A process for the preparation of a hydroxylamine of the formula III

$H_2N$-O-A-Z    III

where

A is a $C_3$-$C_6$-alkynylene chain which is unsubstituted or substituted by 1 to 3 $C_1$-$C_3$-alkyl groups or halogen atoms;

Z is phenyl, or a 5-membered or 6-membered heteroaromatic structure having one or more hetero atoms selected from the group consisting of oxygen, sulfur and nitrogen, where the aromatic radicals may be unsubstituted or substituted by n identical or different radicals X;

X is nitro, cyano, halogen, $C_1$-$C_4$-alkyl, $C_1$-$C_4$-alkoxy, $C_1$-$C_4$-alkylthio, $C_1$-$C_4$-haloalkyl, $C_1$-$C_4$-haloalkoxy, carboxyl, $C_1$-$C_4$-alkoxycarbonyl, benzyloxycarbonyl or phenyl, where the aromatic radicals may carry one to three of the following substituents: nitro, cyano, halogen, $C_1$-$C_4$-alkyl, $C_1$-$C_4$-alkoxy, $C_1$-$C_4$-alkylthio, $C_1$-$C_4$-haloalkyl, $C_1$-$C_4$-haloalkoxy, carboxyl, $C_1$-$C_4$-alkoxycarbonyl, or benzyloxycarbonyl and

n is from 0 to 3, or from 1 to 5 where X is halogen,

wherein a compound of the formula IV

X-A-Z    IV

where A and Z have the abovementioned meanings and X is a nucleophilically substitutable leaving group, is reacted with a cyclic hydroximide of the formula V

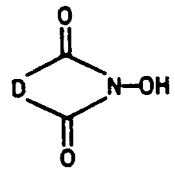

V,

where D is $C_2$- or $C_3$-alkylene, $C_2$-alkenylene or a five-membered or six-membered ring which may contain 1 to 3 double bonds and one nitrogen atom, in the presence of a base to give a compound of the formula VI

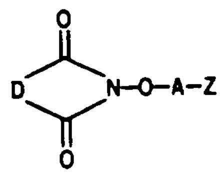

VI,

and the hydroxylamine III is liberated from the imide ether VI by means of a base.

**5.** A process as claimed in claim 4, wherein the hydroxylamine III is liberated from the imide ether VI by means of ethanolamine.

**Revendications**
**Revendications pour les Etats contractants suivants : AT, BE, CH, DE, FR, GB, IT, LI, NL**

**1.** Ethers de cyclohexénonoximes de formule générale I

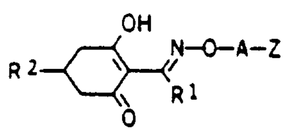

(I)

dans laquelle les symboles ont les significations suivantes :

$R^1$ représente un groupe en C1-C6 ;

A représente une chaîne alcynylène en C3-C6 non substituée ou substituée par 1 à 3 groupes alkyle en C1-C3 ou atomes d'halogènes ;

Z représente un groupe phényle, un groupe hétéroaromatique à 5 ou 6 chaînons contenant 1 à 3 hétéroatomes choisis dans le groupe consistant en trois atomes d'azote et un atome d'oxygène ou de soufre, les radicaux aromatiques pouvant être non substitués ou porter n substituants identiques ou différents X ;

X représente un groupe nitro, cyano, un halogéne, un groupe alkyle en C1-C4, alcoxy en C1-C4, alkylthio en C1-C4, halogénoalkyle en C1-C4, halogénoalcoxy en C1-C4, carboxyle, (alcoxy en C1-C4)-carbonyle, benzyloxycarbonyle, phényle, les radicaux aromatiques pouvant encore porter 1 à 3 substituants choisis parmi les groupes nitro, cyano, les halogènes, les groupes alkyle en C1-C4, alcoxy en C1-C4, alkylthio en C1-C4, halogéno-alkyle en C1-C4, halogénoalcoxy en C1-C4, carboxyle, (alcoxy en C1-C4)-carbonyle et benzyloxycarbonyle ;

n est un nombre allant de 0 à 3 ou de 1 à 5 lorsque X représente des atomes d'halogènes ;

$R^2$ représente un groupe (alcoxy en C1-C4)-alkyle en C1-C6 (alkylthio en C1-C4)-alkyle en C1-C6 ;

un groupe cycloalkyle en C3-C7 ou cycloalcényle en C5-C7, ces groupes pouvant encore porter un à trois substituants choisis parmi les groupes alkyle en C1-C4, alcoxy en C1-C4, alkylthio en C1-C4, halogénoalkyle en C1-C4, les groupes hydroxy et les halogènes ;

un hétérocycle saturé à cinq chaînons contenant un ou deux hétéroatomes choisis dans le groupe consistant en l'oxygène et le soufre, et qui peut encore porter un à trois substituants

choisis parmi les groupes alkyle en C1-C4, alcoxy en C1-C4, alkylthio en C1-C4 et halogénoalkyle en C1-C4,
un hétérocycle saturé ou mono- ou di-insaturé à 6 ou 7 chaînons contenant un ou deux hétéroatomes choisis dans un groupe consistant en l'oxygène et le soufre, l'hétérocycle pouvant encore porter un à trois substituants choisis parmi les groupes hydroxy, les halogènes, les groupes alkyle en C1-C4, alcoxy en C1-C4, alkylthio en C1-C4 et halogénoalkyle en C1-C4 ;
un radical hétéroaromatique à 5 chaînons contenant un à trois hétéroatomes choisis dans un groupe consistant en deux atomes d'azote et un atome d'oxygène ou de soufre, l'hétérocycle pouvant encore porter un à trois substituants choisis parmi les halogènes, les groupes cyano, alkyle en C1-C4, alcoxy en C1-C4, alkylthio en C1-C4, halogénoalkyle en C1-C4, alcényle en C2-C6, alcényloxy en C2-C6, halogénoalcényle en C2-C6 et (alcoxy en C1-C4)-alkyle en C1-C4,
le groupe phényle ou le groupe pyridyle, ces groupes pouvant encore porter un à trois substituants choisis parmi les halogènes, les groupes nitro, cyano, alkyle en C1-C4, alcoxy en C1-C4, alkylthio en C1-C4, halogénoalkyle en C1-C4, alcényloxy en C3-C6, alcynyloxy en C3-C6 et $-NR^4R^4$ dans lequel
$R^3$ représente l'hydrogène, un groupe alkyle en C1-C4, alcényle en C3-C6 ou alcynyle en C3-C6 et
$R^4$ représente l'hydrogène, un groupe alkyle en C1-C4, alcényle en C3-C6, alcynyle en C3-C6, acyle en C1-C6 ou benzoyle dont le noyau aromatique peut porter en outre un à trois substituants choisis parmi les groupes nitro, cyano, les halogènes, les groupes alkyle en C1-C4, alcoxy en C1-C4, alkylthio en C1-C4 et halogénoalkyle en C1-C4,
et leurs sels et esters d'acides carboxyliques en C1-C10 et d'acides inorganiques acceptables pour des applications agricoles.

**2.** Procédé de préparation des composés de formule I de la revendication 1, caractérisé en ce que l'on fait réagir une cyclohexénone de formule II

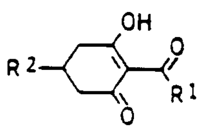

II

de manière connue en soi, dans un solvant organique inerte, avec une hydroxylamine de formule III

$H_2N-O-A-Z$  III

ou avec un sel de cet hydroxylamine, et, le cas échéant, on convertit le produit obtenu, I, en un sel ou un ester d'acide carboxylique en C1-C10 ou d'acide inorganique acceptable pour les usages agricoles.

**3.** Produit herbicide contenant des additifs inertes et une quantité herbicide efficace d'au moins un composé de formule I de la revendication 1.

**4.** Procédé pour combattre les croissances de végétaux indésirables, caractérisé en ce que l'on traite les végétaux indésirables et/ou leur habitat par une quantité herbicide efficace d'un dérivé de cyclohexénone de formule I de la revendication 1.

**5.** Hydroxylamines de formule III

$H_2N-O-A-Z$  III,

dans laquelle A et Z ont les significations suivantes :

A représente une chaîne alcynylène en C3-C6 non substituée ou substituée par un à trois groupes alkyle en C1-C3 ou atomes d'halogènes ;

Z représente un groupe phényle, un groupe hétéroaromatique à 5 ou 6 chaînons contenant un

ou plusieurs hétéroatomes du groupe formé par l'oxygène, le soufre ou l'azote, l'es radicaux aromatiques pouvant être non substitués ou porter n substituants X identiques ou différents;

X représente un groupe nitro, cyano, un halogéne, un groupe alkyle en C1-C4, alcoxy en C1-C4, alkylthio en C1-C4, halogénoalkyle en C1-C4, halogénoalcoxy en C1-C4, carboxyle, (alcoxy en C1-C4)-carbonyle, benzyloxycarbonyle et/ou phényle, les radicaux aromatiques pouvant porter un à trois des substituants suivants : les groupes nitro, cyano, les halogènes, les groupes alkyle en C1-C4, alcoxy en C1-C4, alkylthio en C1-C4, halogéno-alkyle en C1-C4, halogénoalcoxy en C1-C4, carboxyle, (alcoxy en C1-C4)-carbonyle et/ou benzyloxycarbonyle ;

n est un nombre allant de 0 à 3 ou de 1 à 5 lorsque X représente un halogène.

**6.** Procédé de préparation des hydroxylamines de formule III de la revendication 5, caractérisé en ce que l'on fait réagir un composé de formule IV

X-A-Z (IV),

dans laquelle A et Z ont les significations indiquées dans la revendication 5 et X représente un groupe éliminable nucléophile, avec un hydroxyimide cyclique de formule V

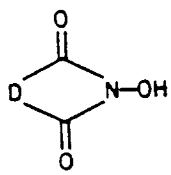

V,

dans laquelle D représente un groupe alkylène en C2-C3, alcénylène en C2 ou un cycle à 5 ou 6 chaînons qui peut contenir une à trois doubles liaisons et un atome d'azote, en présente d'une base, la réaction donnant des composés de formule VI

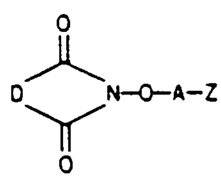

VI,

c'est-à-dire des imidoéthers d'où l'on libère les hydroxylamines III à l'aide de bases.

**7.** Procédé selon la revendication 6, caractérisé en ce que l'on libère l'hydroxylamine III de l'imidoéther VI à l'aide de l'éthanolamine.

**Revendications pour l'Etat contractant suivant : ES**

**1.** Procéde, de préparation des éthers de cyclohexénonoximes de formule générale I

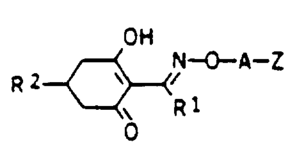

(I)

dans laquelle les symboles ont les significations suivantes :

$R^1$ représente un groupe en C1-C6;

A représente une chaîne alcynylène en C3-C6 non substituée ou substituée par 1 à 3 groupes alkyle en C1-C3 ou atomes d'halogènes ;

Z représente un groupe phényle, un groupe hétéroaromatique à 5 ou 6 chaînons contenant 1 à 3 hétéroatomes choisis dans un groupe consistant en trois atomes d'azote et un atome d'oxygène ou de soufre, les groupes aromatiques pouvant être non substitués ou porter n substituants identiques ou différents X ;

X représente un groupe nitro, cyano, un halogéne, un groupe alkyle en C1-C4, alcoxy en C1-C4, alkylthio en C1-C4, halogénoalkyle en C1-C4, halogénoalcoxy en C1-C4, carboxyle, (alcoxy en C1-C4)-carbonyle, benzyloxycarbonyle, phényle, les groupes aromatiques pouvant encore porter 1 à 3 substituants choisis parmi les groupes nitro, cyano, les halogènes, les groupes alkyle en C1-C4, alcoxy en C1-C4, alkylthio en C1-C4, halogéno-alkyle en C1-C4, halogénoalcoxy en C1-C4, carboxyle, (alcoxy en C1-C4)-carbonyle et benzyloxycarbonyle ;

n est un nombre allant de 0 à 3 ou de 1 à 5 lorsque X représente des atomes d'halogènes ;

$R^2$ représente un groupe (alcoxy en C1-C4)-alkyle en C1-C6 ou (alkylthio en C1-C4)-alkyle en C1-C6 ;

un groupe cycloalkyle en C3-C7 ou cycloalcényle en C5-C7, ces groupes pouvant encore porter un à trois substituants choisis parmi les groupes alkyle en C1-C4, alcoxy en C1-C4, alkylthio en C1-C4, halogénoalkyle en C1-C4, les groupes hydroxy et les halogènes ;

un hétérocycle saturé à cinq chaînons contenant un ou deux hétéroatomes choisis dans un groupe consistant en l'oxygène et le soufre, et qui peut encore porter un à trois substituants choisis parmi les groupes alkyle en C1-C4, alcoxy en C1-C4, alkylthio en C1-C4 et halogénoalkyle en C1-C4,

un hétérocycle saturé ou mono- ou di-insaturé à 6 ou 7 chaînons contenant un ou deux hétéroatomes choisis dans un groupe consistant en l'oxygène et le soufre, l'hétérocycle pouvant encore porter un à trois substituants choisis parmi les groupes hydroxy, les halogènes, les groupes alkyle en C1-C4, alcoxy en C1-C4, alkylthio en C1-C4 et halogéno-alkyle en C1-C4 ;

un groupe hétéroaromatique à 5 chaînons contenant un à trois hétéroatomes choisis dans un groupe consistant en deux atomes d'azote et un atome d'oxygène ou de soufre, l'hétérocycle pouvant encore porter un à trois substituants choisis parmi les halogènes, les groupes cyano, alkyle en C1-C4, alcoxy en C1-C4, alkylthio en C1-C4, halogénoalkyle en C1-C4, alcényle en C2-C6, alcényloxy en C2-C6, halogénoalcényle en C2-C6 et (alcoxy en C1-C4)-alkyle en C1-C4,

le groupe phényle ou le groupe pyridyle, ces groupes pouvant encore porter un à trois substituants choisis parmi les halogènes, les groupes nitro, cyano, alkyle en C1-C4, alcoxy en C1-C4, alkylthio en C1-C4, halogénoalkyle en C1-C4, alcényloxy en C3-C6, alcynyloxy en C3-C6 et $-NR^3R^4$ dans lequel

$R^3$ représente l'hydrogène, un groupe alkyle en C1-C4, alcényle en C3-C6 ou alcynyle en C3-C6 et

$R^4$ représente l'hydrogène, un groupe alkyle en C1-C4, alcényle en C3-C6, alcynyle en C3-C6, acyle en C1-C6 ou benzoyle dont le noyau aromatique peut en outre porter un à trois substituants choisis parmi les groupes nitro, cyano, les halogènes, les groupes alkyle en C1-C4, alcoxy en C1-C4, alkylthio en C1-C4 et halogénoalkyle en C1-C4,

et de leurs sels et esters d'acides carboxyliques en C1-C10 et d'acides inorganiques acceptables pour des usages agricoles,

caractérisé en ce que l'on fait réagir une cyclo-hexénone de formule II

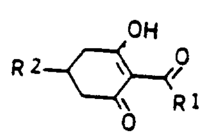

II

de manière connue en soi, dans un solvant organique inerte, avec une hydroxylamine de formule III

$H_2N-O-A-Z$ III,

ou avec un sel de cet hydroxylamine, et le cas échéant, on convertit le produit obtenu, de formule I, en un sel ou ester d'acide carboxylique en C1-C10 ou d'acide inorganique acceptable pour les usages

agricoles.

**2.** Produit herbicide contenant des additifs inertes et une quantité herbicide efficace d'au moins un composé de formule I de la revendication 1.

**3.** Procédé pour combattre les croissances de végétaux indésirables, caractérisé en ce que l'on traite les végétaux indésirables et/ou leur habitat par une quantité herbicide efficace d'un dérivé de cyclohexénone de formule I de la revendication 1.

**4.** Procédé de préparation des hydroxylamines de formule III

$H_2N$-O-A-Z (III)

dans laquelle A et Z ont les significations suivantes :

A représente une chaîne alcynylène en C3-C6 non substituée par un à trois groupes alkyle en C1-C3 ou atomes d'halogènes ;

Z représente un groupe phényle, un groupe hétéroaromatique à 5 ou 6 chaînons contenant un ou plusieurs hétéroatomes choisis parmi l'oxygène, le soufre ou l'azote, les groupes aromatiques pouvant être non substitués ou porter n substituants identiques ou différents X ;

X représente un groupe nitro, cyano, un halogéne, un groupe alkyle en C1-C4, alcoxy en C1-C4, alkylthio en C1-C4, halogénoalkyle en C1-C4, halogénoalcoxy en C1-C4, carboxyle, (alcoxy en C1-C4)-carbonyle, benzyloxycarbonyle et/ou phényle, les groupes aromatiques pouvant porter un à trois des substituants suivants : les groupes nitro, cyano, les halogènes, les groupes alkyle en C1-C4, alcoxy en C1-C4, alkylthio en C1-C4, halogénoalkyle en C1-C4, halogénoalcoxy en C1-C4, carboxyle, (alcoxy en C1-C4)-carbonyle et/ou benzyloxy-carbonyle ;

n est un nombre allant de 0 à 3 ou de 1 à 5 lorsque X représente un halogène,

caractérisé en ce que l'on fait réagir un composé de formule IV

X-A-Z IV,

dans laquelle A et Z ont les significations indiquées dans la revendication 1, et X représente un groupe éliminable nucléophile, avec un hydroxyimide cyclique de formule V

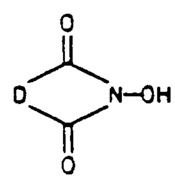

V,

dans laquelle D représente un groupe alkylène en C2-C3, alcénylène en C2 ou un cycle à 5 ou 6 chaînons qui peut contenir une à trois doubles liaisons et un atome d'azote, en présence d'une base, la réaction donnant des composés de formule VI

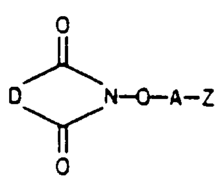

VI,

c'est-à-dire des imidoéthers VI d'où l'on libère les hydroxylamines III à l'aide de bases.

**5.** Procédé selon la revendication 4, caractérisé en ce qu'on libère l'hydroxylamine III de l'imidoéther VI à l'aide de l'éthanolamine.